# EUROPEAN PATENT APPLICATION

(11) **EP 1 270 726 A1**
(43) Date of publication of application: **02.01.2003**
(21) Application number: 02254232.8
(22) Date of filing: 18.06.2002
(51) Int. Cl.: C12N 15/12, C07K 14/705, C07K 16/28, C12Q 1/68, G01N 33/50

(54) **69624, a human transporter family member and uses therefor**

(30) Priority: 18.06.2001 US 298970 P
(71) Applicant: Millennium Pharmaceuticals, Inc., Cambridge, MA 02139 (US)
(72) Inventor: Curtis, Rory A J, Framingham, Massachusetts 01702 (US)
(74) Representative: Lunt, Mark George Francis

(57) **Abstract**

The invention provides isolated nucleic acids molecules, designated 69624 nucleic acid molecules, which encode novel transporter members. The invention also provides antisense nucleic acid molecules, recombinant expression vectors containing 69624 nucleic acid molecules, host cells into which the expression vectors have been introduced, and nonhuman transgenic animals in which a 69624 gene has been introduced or disrupted. The invention still further provides isolated 69624 proteins, fusion proteins, antigenic peptides and anti-69624 antibodies. Diagnostic methods utilizing compositions of the invention are also provided.

## Description

### Background of the Invention

Cellular membranes serve to differentiate the contents of a cell from the surrounding environment. These membranes may also serve as effective barriers against the unregulated influx of hazardous or unwanted compounds, and the unregulated efflux of desirable compounds. However, the cell does need a supply of desired compounds and the removal of waste products. Transport proteins which are embedded (singly or in complexes) in the cellular membrane (reviewed by Oh and Amidon (1999) in Membrane Transporters as Drug Targets, ed. Amidon and Sadee, Kluwer Academic/Plenum Publishers, New York, Chapter 1) are major providers of these functions. There are two general classes of membrane transport proteins: channels or pores, and transporters (also known as carriers or permeases). Channels and transporters differ in their translocation mechanisms. Channels are hydrophilic group-lined protein tunnels whose opening by a regulatory event allows free, rapid passage of their charge-, size-, and geometry-selected small ions down their concentration gradients. Transporters specifically and selectively bind the molecules they move, some with and some against their concentration gradients, across membranes. The binding mechanism causes the action of transporters to be slow and saturable.

Transport molecules are specific for a particular target solute or class of solutes, and are also present in one or more specific membranes. Transport molecules localized to the plasma membrane permit an exchange of solutes with the surrounding environment, while transport molecules localized to intracellular membranes (e.g., membranes of the mitochondrion, peroxisome, lysosome, endoplasmic reticulum, nucleus, or vacuole) permit import and export of molecules from organelle to organelle or to the cytoplasm. For example, in the case of the mitochondrion, transporters in the inner and outer mitochondrial membranes permit the import of sugar molecules, calcium ions, and water (among other molecules) into the organelle and the export of newly synthesized ATP to the cytosol.

Transporters can move molecules by two types of processes. In one process, "facilitated diffusion," transporters move molecules with their concentration gradients. In the other process, "active transport," transporters move molecules against their concentration gradients. To move a molecule against its concentration gradient requires energy. Primary active transporters, such as Na+/K+ ATPases or ABC transporters, use energy from ATP hydrolysis or light to establish ion gradients and create membrane potentials. Secondary active transporters, such as the H+/peptide transporter, use the energy stored in pH or ion gradients (resulting from the actions of primary active transporters) to transport other molecules. In secondary active transport, the transporter uses two separate binding sites: one to move the primary ion down its concentration gradient; and another to move the secondary solute against its gradient, using the energy released by the movement of the primary ion. The coupled solute either travels in the same direction as the primary solute (symport) or in the opposite direction (antiport).

Transporters play important roles in the ability of the cell to regulate homeostasis, to grow and divide, and to communicate with other cells, e.g., to transport signaling molecules, such as hormones, reactive oxygen species, ions, neurotransmitters or vitamins. A wide variety of human diseases and disorders are associated with defects in transporter or other membrane transport molecules, including certain types of liver disorders (e.g., due to defects in transport of long-chain fatty acids (Al Odaib *et al.* (1998) *New Eng. J. Med.* 339:1752-1757), hyperlysinemia (mitochondrial lysine transport defect (Oyanagi *et al.* (1986) *Inherit. Metab. Dis.* 9:313-316)), and cataract (Wintour (1997) *Clin. Exp.*

### Pharmacol. Physiol. 24(1):1-9).

There are over 30 families of secondary transporters, also known as solute carriers or SLCs (reviewed by Berger, *et al.* (2000) in The Kidney: Physiology and Pathophysiology, eds. Seldin DW and Giebisch G., Lippincott, Williams & Wilkins, Philadelphia 1:107-138; see also www.gene.ucl.ac.uk/nomenclature). The SLC families are classified according to the pair of molecules they move. The SLC13 family couples the transport of dicarboxylate or sulfate ions to the transport of sodium ions (Chen *et al.* (1998), *J Biol Chem* 273(33):20972-81). SLC13 family members are expressed in a number of tissues, including kidney, intestine, lung, liver, and brain (Chen et al. (1999), *J Clin Invest* 103(8):1159-68; Huang *et al.* (2000), *J Pharmacol Exp Ther* 295(1):392-403).

### Summary of the Invention

The present invention is based, in part, on the discovery of a novel transporter family member, referred to herein as "69624". The nucleotide sequence of a cDNA encoding 69624 is recited in SEQ ID NO:1, and the amino acid sequence of a 69624 polypeptide is recited in SEQ ID NO:2. In addition, the nucleotide sequences of the coding region are recited in SEQ ID NO:3.

Accordingly, in one aspect, the invention features an isolated nucleic acid molecule that encodes a 69624 protein or polypeptide, e.g., a biologically active portion of the 69624 protein. In a preferred embodiment the isolated nucleic acid molecule encodes a polypeptide having the amino acid sequence of SEQ ID NO:2. In other embodiments, the invention provides isolated 69624 nucleic acid molecules having the nucleotide sequence shown in SEQ ID NO:1 or SEQ ID NO:3. In still other embodiments, the invention provides isolated nucleic acid molecules that are substantially identical (e.g., at least 80%, 85%, 90%, 95%, 96%, 97%, 98% or 99% identical or which are naturally occurring allelic variants) to the nucleotide sequence shown in SEQ ID NO:1 or SEQ ID NO:3. In other embodiments, the invention provides an isolated nucleic acid molecule which hybridizes under a stringency condition described herein to a nucleic acid molecule comprising the nucleotide sequence of SEQ ID NO: 1 or SEQ ID NO:3, wherein the nucleic acid encodes a full length 69624 protein or an active fragment thereof. In yet another embodiment, the isolated nucleic acid molecule encodes a fragment of a polypeptide comprising the amino acid sequence of SEQ ID NO:2, wherein the fragment comprises at least 150 contiguous amino acids of SEQ ID NO:2. In another embodiment, the nucleic acid molecule encodes an antigenic peptide or fragment of a polypeptide comprising the amino acid sequence of SEQ ID NO:2, wherein the fragment comprises at least 8 contiguous amino acids of SEQ ID NO:2. In another embodiment, the nucleic acid molecule encodes a naturally occurring allelic variant of a polypeptide comprising the amino acid sequence of SEQ ID NO:2, wherein the nucleic acid molecule hybridizes to a nucleic acid molecule comprising SEQ ID NO:1, 3, or a complement thereof, under a stringent condition as described herein. In still another embodiment, an isolated nucleic acid described here can further include a heterologous sequence.

In a related aspect, the invention further provides nucleic acid constructs that include a 69624 nucleic acid molecule described herein. In certain embodiments, the nucleic acid molecules of the invention are operatively linked to native or heterologous regulatory sequences. Also included, are vectors and host cells containing the 69624 nucleic acid molecules of the invention e.g., vectors and host cells suitable for producing 69624 nucleic acid molecules and polypeptides.

In another related aspect, the invention provides isolated nucleic acid fragments suitable as primers or hybridization probes for the detection of 69624-encoding nucleic acids. In one embodiment, the isolated nucleic acid fragment is at least 300, 400, 500, 600 or more nucleotides of the nucleotide sequence of SEQ ID NO:1 or SEQ ID NO:3. In still another related aspect, isolated nucleic acid molecules that are antisense to a 69624 encoding nucleic acid molecule are provided.

In another aspect, the invention features, isolated 69624 polypeptides, and biologically active or antigenic fragments thereof that are useful, e.g., as reagents or targets in assays applicable to treatment and diagnosis of 69624-mediated or -related disorders. In another embodiment, the invention provides 69624 polypeptides having a 69624 activity. Preferred polypeptides are 69624 proteins including at least one transporter domain, e.g., a sodium-dependent dicarboxylate/sulfate transporter domain, and, preferably, having a 69624 activity, e.g., a 69624 activity as described herein.

In other embodiments, the invention provides isolated 69624 polypeptides, e.g., an isolated 69624 polypeptide chosen from one of the following: (i) the amino acid sequence shown in SEQ ID NO:2; (ii) an amino acid sequence that is substantially identical (e.g., at least 80%, 85%, 90%, 95%, 96%, 97%, 98%, or 99% identical) to the amino acid sequence shown in SEQ ID NO:2; (iii) a naturally occurring allelic variant of a polypeptide comprising the amino acid sequence of SEQ ID NO:2, wherein the polypeptide is encoded by a nucleic acid molecule which hybridizes to a nucleic acid molecule comprising SEQ ID NO:1, SEQ ID NO:3, or a complement thereof under stringent conditions as described herein; (iv) a fragment of a polypeptide comprising the amino acid sequence of SEQ ID NO:2, wherein the fragment comprises at least 15 contiguous amino acids of SEQ ID NO:2; or (v) an amino acid sequence encoded by a nucleic acid molecule having a nucleotide sequence which hybridizes under a stringency condition described herein to a nucleic acid molecule comprising the nucleotide sequence of SEQ ID NO: 1 or SEQ ID NO:3, wherein the nucleic acid encodes a full length 69624 protein or an active fragment thereof.

In a related aspect, the invention provides 69624 polypeptides or fragments operatively linked to a heterologous amino acid sequence, e.g., a non-69624 polypeptide to form fusion proteins.

In another aspect, the invention features antibodies and antigen-binding fragments thereof, that react with, or more preferably specifically bind 69624 polypeptides or fragments thereof, e.g., a transporter domain, e.g., an extracellular epitope of a 69624 transporter domain. In one embodiment, the antibodies or antigen-binding fragment thereof competitively inhibit the binding of a second antibody to 69624 polypeptides or fragments thereof, e.g., a transporter domain, e.g., an extracellular epitope of a 69624 transporter domain.

In another aspect, methods for producing a 69624 polypeptide, e.g., a 69624 polypeptide as described herein, are provided. The method includes introducing a 69624 nucleic acid into a host cell, e.g., a host cell as described herein, and culturing the host cell under conditions in which the nucleic acid molecule is expressed, thereby producing the 69624 polypeptide.

In another aspect, the invention provides a method of identifying or screening for an agent (e.g., a compound) that modulates the expression or activity of the 69624 polypeptides or nucleic acids. The method includes contacting a 69624 polypeptide or nucleic acid as described herein, or a cell expressing a 69624 polypeptide or nucleic acid as described herein with a test agent (e.g., a test compound), and determining whether the test agent binds to, or modulates the expression or activity of a 69624 polypeptide or nucleic acid. For example, the binding of the test agent to the polypeptide can be detected by one of the following methods: (i) detection of binding by direct detection of test agent/polypeptide binding; (ii) detection of binding using a competition binding assay; or (iii) detection of binding using an assay for 69624-mediated signal transduction.

In still another aspect, the invention provides a process for modulating 69624 polypeptide or nucleic acid expression or activity, e.g. using the screened compounds. In certain embodiments, the methods involve treatment of conditions related to aberrant activity or expression of the 69624 polypeptides or nucleic acids, such as conditions involving aberrant or deficient renal, hepatic, or neural function.

The invention also provides assays for determining the activity of or the presence or absence of 69624 polypeptides or nucleic acid molecules in a biological sample, including for disease diagnosis.

In further aspect, the invention provides assays for determining the presence or absence of a genetic alteration in a 69624 polypeptide or nucleic acid molecule, including for disease diagnosis.

In another aspect, the invention features a two dimensional array having a plurality of addresses, each address of the plurality being positionally distinguishable from each other address of the plurality, and each address of the plurality having a unique capture probe, e.g., a nucleic acid or peptide sequence. At least one address of the plurality has a capture probe that recognizes a 69624 molecule. In one embodiment, the capture probe is a nucleic acid, e.g., a probe complementary to a 69624 nucleic acid sequence. In another embodiment, the capture probe is a polypeptide, e.g., an antibody specific for 69624 polypeptides. Also featured is a method of analyzing a sample by contacting the sample to the aforementioned array and detecting binding of the sample to the array.

Other features and advantages of the invention will be apparent from the following detailed description, and from the claims.

### Brief Description of the Drawing

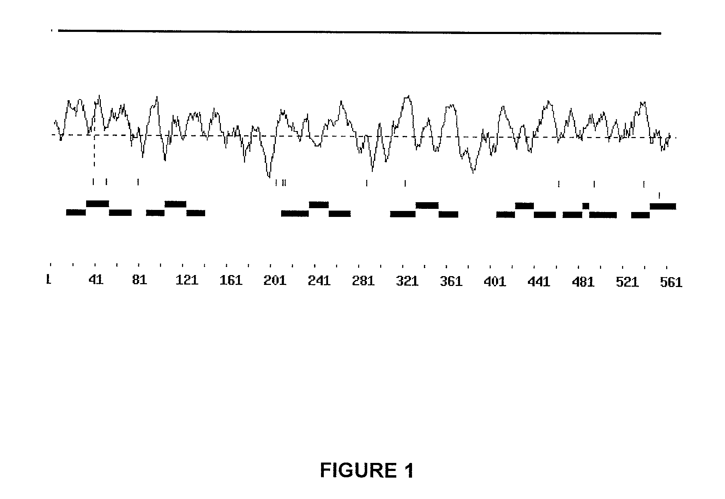

*Figure 1* depicts a hydropathy plot of human 69624. Relative hydrophobic residues are shown above the dashed horizontal line, and relative hydrophilic residues are below the dashed horizontal line. The cysteine residues (cys) are indicated by short vertical lines just below the hydropathy trace. The numbers corresponding to the amino acid sequence of human 69624 are indicated. Polypeptides of the invention include fragments which include: all or part of a hydrophobic sequence, i.e., a sequence above the dashed line, e.g., the sequence from about amino acid 14 to31, from about 253 to 272, and from about 309 to 331 of SEQ ID NO:2; all or part of a hydrophilic sequence, i.e., a sequence below the dashed line, e.g., the sequence of from about amino acid 189 to 206, from about 290 to 308, and from about 372 to 388 of SEQ ID NO:2; a sequence which includes a Cys, or a glycosylation site.

### Detailed Description

The human 69624 sequence (SEQ ID NO:1), which is approximately 2534 nucleotides long including untranslated regions, contains a predicted methionine-initiated coding sequence of about 1707 nucleotides, including the termination codon (nucleotides indicated as coding of SEQ ID NO:1; SEQ ID NO:3). The coding sequence encodes a 568 amino acid protein (SEQ ID NO:2).

Human 69624 contains the following regions or other structural features:

a sodium sulfate symporter domain (PFAM Accession Number PF00939, SEQ ID NO:4) located at about amino acid residues 6 to 554 of SEQ ID NO:2;

a sodium sulfate symporter signature motif (PS01271, SEQ ID NO:6) located at about amino acid residues 498 to 514 of SEQ ID NO:2;

thirteen transmembrane domains (predicted by MEMSAT, Jones *et al.* (1994) *Biochemistry* 33:3038-3049) located at about amino acid residues 14 to 31, 53 to 73, 87 to 103, 124 to 140, 210 to 234, 253 to 272, 309 to 331, 353 to 370, 406 to 422, 440 to 459, 466 to 483, 490 to 514, and 528 to 544 of SEQ ID NO:2;

two Protein Kinase C phosphorylation sites (PS00005) located at about amino acid residues 291 to 293, and 372 to 374 of SEQ ID NO:2;

two Casein Kinase II phosphorylation sites (PS00006) located at about amino acids 381 to 384, and 564 to 567 of SEQ ID NO:2;

one N-glycosylation site (PS00001) located at about amino acids 562 to 565 of SEQ ID NO:2; and

three N-myristoylation sites (PS00008) located at about amino acids 118 to 123, 226 to 231, and 423 to 428 of SEQ ID NO:2.

For general information regarding PFAM identifiers, PS prefix and PF prefix domain identification numbers, refer to Sonnhammer *et al.* (1997) *Protein* 28:405-420 and http://www.psc.edu/general/ software/packages/pfam/pfam.html.

The 69624 protein contains a significant number of structural characteristics in common with members of the SLC13 sodium dicarboxylate/sulfate transporter family. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologues of non-human origin, e.g., rat or mouse proteins. Members of a family can also have common functional characteristics.

Sodium dicarboxylate/sulfate transporter family members are characterized by a common fold. At present, sodium dicarboxylate/sulfate transporter domains are believed to have at least eleven transmembrane domains, with the possibility of there being twelve or even thirteen transmembrane domains. It is also believed that the N-terminal non-transmembrane domain is located in the cytoplasm, while the C-terminal non-transmembrane domain is located extracellularly, although confirmation of these beliefs awaits further evidence (see Pajor and Sun (1996), *Am J Physiol Cell Physiol* 271:C1808-16 and Wang *et al.* (2000) *Am J Physiol Cell Physiol* 278:C1019-30, the contents of which are incorporated herein by reference). The analysis of chimeric sodium dicarboylate/sulfate transporters has suggested that substrate specificity is encoded in the C-terminal half of the sodium dicarboxylate/sulfate transporter domain (see Chen *et al.* (2000), *J Clin Invest* 103:1159-68, the contents of which are incorporated herein by reference). The 69624 polypeptide shares homology with members of the SLC13 sodium dicarboxylate/sulfate transporter family. A GAP alignment of human 69624 with a member of the SLC13 sodium dicarboxylate/sulfate transporter family, rat NaDC-1, a sodium-dependent dicarboxylate/sulfate transporter (SEQ ID NO:5; Genbank accession number AB001321) shows 54.7% identity (as determined from a matrix made by matblas from blosum62.iij).

A 69624 polypeptide can include a "sodium dicarboxylate/sulfate transporter domain" or regions homologous with a "sodium dicarboxylate/sulfate transporter domain".

As used herein, the term "sodium dicarboxylate/sulfate transporter domain" includes an amino acid sequence of about 450 to 650 amino acid residues in length and having a bit score for the alignment of the sequence to the sodium sulfate symporter domain profile (Pfam HMM, SEQ ID NO:4) of at least 250. Preferably, a sodium dicarboxylate/sulfate transporter domain includes at least about 500 to 625 amino acids, more preferably about 525 to 610 amino acid residues, or about 550 to 600 amino acids and has a bit score for the alignment of the sequence to the sodium sulfate symporter domain (HMM) of at least 350, 400, 450 or greater. The sodium sulfate symporter domain (HMM) has been assigned the PFAM Accession Number PF00939 (http;//genome.wustl.edu/Pfam/.html). An alignment of the sodium dicarboxylate/sulfate domain (amino acids 6 to 554 of SEQ ID NO:2) of human 69624 with a consensus amino acid sequence (SEQ ID NO:4) derived from a hidden Markov model yields a bit score of 488.2.

In a preferred embodiment a 69624 polypeptide or protein has a "sodium dicarboxylate/sulfate transporter domain" or a region which includes at least about 500 to 625 more preferably about 525 to 610, or 550 to 600 amino acid residues and has at least about 60%, 70% 80% 90% 95%, 99%, or 100% homology with a "sodium dicarboxylate/sulfate transporter domain," e.g., the sodium dicarboxylate/sulfate transporter domain of human 69624 (e.g., residues 6 to 554 of SEQ ID NO:2).

In the sodium dicarboxylate/sulfate domain are subdomains which have homology to portions of ProDom cotransporter sodium/dicarboxylate domains. As used herein, a "cotransporter sodium/dicarboxylate domain" is a portion of the human 69624 protein which is homologous, *e.g.*, at least about 54%, 55%, 56%, 57%, 58%, 59%, 60%, 61%, 62%, 63%, 64%, 65%, 66%, 67%, 68%, 69%, 70%, 71%, 72%, 73%, or 74% identical to the ProDom family PD084877 "Transport Cotransporter Transmembrane Sodium Symport Na/Dicarboxylate Sodium/Dicarboxylate Chromosome" domain (ProDomain Release 2001.1; http://www.toulouse.inra.fr/prodom.html, SEQ ID NO:7) or to the ProDom family PD002007 "Transport Cotransporter Transmembrane Sodium Na/Dicarboxylate Symport Sodium/Dicarboxylate Chromosome" domain (ProDomain Release 2001.1; http://www.toulouse.inra.fr/prodom.html, SEQ ID NO:8). An alignment of the first cotransporter sodium/dicarboxylate domain (amino acids 41 to 167 of SEQ ID NO:2) of human 69624 with the sequence selected from the consensus, SEQ ID NO:7, derived from a BLAST search model shows 65% identity (as calculated in ProDomain from the blosum62 matrix). An alignment of the second cotransporter sodium/dicarboxylate domain (amino acids 202 to 375 of SEQ ID NO:2) of human 69624 with the sequence selected from the consensus, SEQ ID NO:8 derived from a BLAST search model shows 59% identity (as calculated in ProDomain from the blosum62 matrix).

To identify the presence of a "sodium dicarboxylate/sulfate transporter" domain in a 69624 protein sequence, and make the determination that a polypeptide or protein of interest has a particular profile, the amino acid sequence of the protein can be searched against the PFAM database of HMMs (e.g., the PFAM database, release 2.1) using the default parameters (http://www.sanger.ac.uk/Software/Pfam/HMM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the PFAM database can be found in Sonhammer *et al.* (1997) *Proteins* 28(3):405-420 and a detailed description of HMMs can be found, for example, in Gribskov *et al*.(1990) *Meth. Enzymol.* 183:146-159; Gribskov *et al*.(1987) *Proc. Natl. Acad. Sci. USA* 84:4355-4358; Krogh *et al*.(1994) J *Mol. Biol.* 235:1501-1531; and Stultz *et al*.(1993) *Protein Sci.* 2:305-314, the contents of which are incorporated herein by reference. A search was performed against the HMM database resulting in the identification of a "sodium sulfate symporter" domain in the amino acid sequence of human 69624 at about residues 6 to 554 of SEQ ID NO:2.

Alternatively, to identify the presence of a "cotransporter sodium/dicarboxylate" domain in a 69624 protein sequence, and make the determination that a polypeptide or protein of interest has a particular profile, the amino acid sequence of the protein can be searched against another database of domains, e.g., the ProDom database (Corpet *et al.* (1999), *Nucl. Acids Res.* 27:263-267). The ProDom protein domain database consists of an automatic compilation of homologous domains. Current versions of ProDom are built using recursive PSI-BLAST searches (Altschul SF *et al.* (1997) *Nucleic Acids Res.* 25:3389-3402; Gouzy *et al.* (1999) *Computers and Chemistry* 23:333-340.) of the SWISS-PROT 38 and TREMBL protein databases. The database automatically generates a consensus sequence for each domain. A BLAST search was performed against the ProDom database resulting in the identification of portions of a "cotransporter sodium/dicarboxylate" domain in the amino acid sequence of human 69624 at about residues 41 to 167 of SEQ ID NO:2 and about residues 202 to 375 of SEQ ID NO:2.

In one embodiment, a 69624 protein includes at least one sodium sulfate symporter signature motif, located at about amino acid residues 498 to 514 of SEQ ID NO:2. As used herein, the term "sodium sulfate symporter signature motif' includes a sequence of at least 13 amino acid residues, defined by the sequence: [STACP]-S-X-X-F-X-X-P-[LIVM]-[GSA]-X-X-X-N-X-[LIVM]-V (SEQ ID NO:6). A sodium sulfate symporter signature motif, as defined, can be involved in the transport of sodium and sulfate or dicarboxylate ions, e.g., citrate, succinate, or N-acetylaspartate, across a biological membrane. More preferably, a sodium sulfate symporter signature motif includes 16, or even more preferably, 17 amino acid residues. Sodium sulfate symporter signature motifs have been described, e.g., in the PROSITE database (http://www.expasy.ch/prosite), where they have been given the PROSITE accession number PS01271.

In a preferred embodiment, a 69624 polypeptide has at least one sodium sulfate symporter signature motif, or a region which includes 13, 16, or 17 amino acid residues and has at least about 75%, 85%, 90%, 95%, or 100% homology with a "sodium sulfate symporter signature motif", e.g., the sodium sulfate symporter signature motif of human 69624, e.g., about amino acid residues 498 to 514 of SEQ ID NO:2.

In another embodiment, a 69624 protein includes at least one, two, three, four, five, six, seven, eight, nine, ten, or preferably, eleven, twelve or thirteen transmembrane domains. As used herein, the term "transmembrane domain" includes an amino acid sequence of at least about 15 amino acid residues in length that inserts into or spans a phospholipid membrane. A transmembrane domain, as defined, can anchor a polypeptide to a lipid bilayer. More preferably, a transmembrane domain includes about at least 18, 20, 22, 24, 25, 30, 35 or 40 amino acid residues and spans a phospholipid membrane. Transmembrane domains are rich in hydrophobic residues, and typically have an α-helical structure. In a preferred embodiment, at least 50%, 60%, 70%, 80%, 90%, 95% or more of the amino acids of a transmembrane domain are hydrophobic, e.g., leucines, isoleucines, tyrosines, phenylalanine, valine, methionine, or tryptophans. Transmembrane domains are described in, for example, http://pfam.wustl.edu/cgi-bin/getdesc?name=7tm-1, and Zagotta W.N. *et al,* (1996) *Annual Rev. Neurosci.* 19: 235-63, the contents of which are incorporated herein by reference.

In a preferred embodiment, a 69624 polypeptide or protein has at least one, two, three, four, five, six, seven, eight, nine, ten, and preferably eleven, twelve or even thirteen transmembrane domains or regions which include at least 18, 20, 22, 24, 25, 30, 35 or 40 amino acid residues and have at least about 75%, 85%, 90%, 95%, 99%, or 100% homology with a "transmembrane domain," e.g., at least one transmembrane domain of human 69624 (e.g., amino acid residues 14 to 31, 53 to 73, 87 to 103, 124 to 140, 210 to 234, 253 to 272, 309 to 331, 353 to 370, 406 to 422, 440 to 459, 466 to 483, 490 to 514, and 528 to 544 of SEQ ID NO:2).

A 69624 family member can include at least one sodium dicarboxylate/sulfate transporter domain. Furthermore, a 69624 family member can include: at least one sodium sulfate symporter signature sequence (PS01271); at least one, two, three, four, five, six, seven, eight, nine, ten, preferably eleven, twelve, or even thirteen predicted transmembrane domains; at least one, preferably two predicted protein kinase C phosphorylation sites (PS00005); at least one, preferably two predicted casein kinase II phosphorylation sites (PS00006); at least one, two, preferably three predicted N-myristoylation sites (PS00008); and at least one predicted N-glycosylation site (PS00001).

As the 69624 polypeptides of the invention may modulate 69624-mediated activities, they may be useful for developing novel diagnostic and therapeutic agents for 69624-mediated or related disorders, as described below.

As used herein, a "69624 activity", "biological activity of 69624" or "functional activity of 69624", refers to an activity exerted by a 69624 protein, polypeptide or nucleic acid molecule. For example, a 69624 activity can be an activity exerted by 69624 in a physiological milieu on, e.g., a 69624-responsive cell or on a 69624 substrate, e.g., a protein substrate. A 69624 activity can be determined *in vivo* or *in vitro.* In one embodiment, a 69624 activity is a direct activity, such as an association with a 69624 target molecule. A "target molecule" or "binding partner" is a molecule with which a 69624 protein binds or interacts in nature. In an exemplary embodiment, 69624 is a transporter, e.g., an active transporter, e.g., a sodium dicarboxylate/sulfate transporter.

A 69624 activity can also be an indirect activity, e.g., a cellular signaling activity mediated by the intracellular or extracellular transport of a 69624 substrate. Based on the above-described sequence similarities, the 69624 molecules of the present invention are predicted to have similar biological activities as sodium dicarboxylate/sulfate transporter family members. For example, the 69624 proteins of the present invention can have one or more of the following activities: (1) transport of ions, e.g., sodium ions, across membranes; (2) transport of dicarboxylate ions, e.g., citrate, succinate, or N-acetylaspartate, across membranes; (3) transport of sulfate ions across membranes; (4) regulation of the intracellular concentrations of metabolic intermediates, e.g., citrate and succinate; (5) regulation of the secretion of anions, e.g., organic anions, e.g., xenobiotic anions or drugs, from cells, e.g., kidney cells, brain cells, colon cells, liver cells, or salivary gland cells; (6) regulation of homeostasis, e.g., by resorption of ions, e.g., citrate or sulfate ions, from urine; (7) regulation, e.g., negative regulation, of the formation of calcium-containing deposits in the body, e.g., renal stones; (8) regulation myelin synthesis and neuronal myelination; (9) regulation of hepatic metabolism, e.g., hepatic glutamine and ammonia metabolism; and (10) regulation of cholesterol metabolism.

The 69624 molecules of the invention can modulate the activities of cells in tissues where they are expressed. For example, cDNA clones containing the 69624 molecules of the invention were isolated from kidney, brain, colon, liver, and salivary gland. Accordingly, the 69624 molecules of the invention can act as therapeutic or diagnostic agents for renal, neurological, colon, hepatic, or disorders.

Thus, the 69624 molecules of the salivary gland invention may act as novel diagnostic targets and therapeutic agents for controlling disorders that are known or thought to involve the abnormal transport of anions, e.g., dicarboxylate anions, e.g. citrate, succinate, or N-acetylaspartate, and sulfate anions. Such disorders include disorders associated with abnormal cellular osmotic stress, hypocitranuria, the formation of calcium stones, abnormal N-acetylaspartate transport, abnormal myelin synthesis, mental retardation (e.g., Canavan disease), and abnormal body sulfate homeostasis. Furthermore, 69624 molecules can act as novel diagnostic targets and therapeutic agents for controlling disorders associated with the kidney, brain, colon, liver, salivary gland, as well as metabolic disorders.

Disorders involving the kidney include, but are not limited to, congenital anomalies including, but not limited to, cystic diseases of the kidney, that include but are not limited to, cystic renal dysplasia, autosomal dominant (adult) polycystic kidney disease, autosomal recessive (childhood) polycystic kidney disease, and cystic diseases of renal medulla, which include, but are not limited to, medullary sponge kidney, and nephronophthisisuremic medullary cystic disease complex, acquired (dialysis-associated) cystic disease, such as simple cysts; glomerular diseases including pathologies of glomerular injury that include, but are not limited to, in situ immune complex deposition, that includes, but is not limited to, anti-GBM nephritis, Heymann nephritis, and antibodies against planted antigens, circulating immune complex nephritis, antibodies to glomerular cells, cell-mediated immunity in glomerulonephritis, activation of alternative complement pathway, epithelial cell injury, and pathologies involving mediators of glomerular injury including cellular and soluble mediators, acute glomerulonephritis, such as acute proliferative (poststreptococcal, postinfectious) glomerulonephritis, including but not limited to, poststreptococcal glomerulonephritis and nonstreptococcal acute glomerulonephritis, rapidly progressive (crescentic) glomerulonephritis, nephrotic syndrome, membranous glomerulonephritis (membranous nephropathy), minimal change disease (lipoid nephrosis), focal segmental glomerulosclerosis, membranoproliferative glomerulonephritis, IgA nephropathy (Berger disease), focal proliferative and necrotizing glomerulonephritis (focal glomerulonephritis), hereditary nephritis, including but not limited to, Alport syndrome and thin membrane disease (benign familial hematuria), chronic glomerulonephritis, glomerular lesions associated with systemic disease, including but not limited to, systemic lupus erythematosus, Henoch-Schönlein purpura, bacterial endocarditis, diabetic glomerulosclerosis, amyloidosis, fibrillary and immunotactoid glomerulonephritis, and other systemic disorders; diseases affecting tubules and interstitium, including acute tubular necrosis and tubulointerstitial nephritis, including but not limited to, pyelonephritis and urinary tract infection, acute pyelonephritis, chronic pyelonephritis and reflux nephropathy, and tubulointerstitial nephritis induced by drugs and toxins, including but not limited to, acute drug-induced interstitial nephritis, analgesic abuse nephropathy, nephropathy associated with nonsteroidal anti-inflammatory drugs, and other tubulointerstitial diseases including, but not limited to, urate nephropathy, hypercalcemia and nephrocalcinosis, and multiple myeloma; diseases of blood vessels including benign nephrosclerosis, malignant hypertension and accelerated nephrosclerosis, renal artery stenosis, and thrombotic microangiopathies including, but not limited to, classic (childhood) hemolytic-uremic syndrome, adult hemolytic-uremic syndrome/thrombotic thrombocytopenic purpura, idiopathic HUS/TTP, and other vascular disorders including, but not limited to, atherosclerotic ischemic renal disease, atheroembolic renal disease, sickle cell disease nephropathy, diffuse cortical necrosis, and renal infarcts; urinary tract obstruction (obstructive uropathy); urolithiasis (renal calculi, stones); and tumors of the kidney including, but not limited to, benign tumors, such as renal papillary adenoma, renal fibroma or hamartoma (renomedullary interstitial cell tumor), angiomyolipoma, and oncocytoma, and malignant tumors, including renal cell carcinoma (hypernephroma, adenocarcinoma of kidney), which includes urothelial carcinomas of renal pelvis.

Disorders involving the brain include, but are not limited to, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and low-flow states--global cerebral ischemia and focal cerebral ischemia--infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropod-borne (Arbo) viral encephalitis, *Herpes simplex* virus Type 1, *Herpes simplex* virus Type 2, *Varicalla-zoster* virus (*Herpes zoster*), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, other infectious diseases of the nervous system; transmissible spongiform encephalopathies (prion diseases); demyelinating diseases, including multiple sclerosis, multiple sclerosis variants, acute disseminated encephalomyelitis and acute necrotizing hemorrhagic encephalomyelitis, and other diseases with demyelination; degenerative diseases, such as degenerative diseases affecting the cerebral cortex, including Alzheimer disease and Pick disease, degenerative diseases of basal ganglia and brain stem, including Parkinsonism, idiopathic Parkinson disease (paralysis agitans), progressive supranuclear palsy, corticobasal degenration, multiple system atrophy, including striatonigral degenration, Shy-Drager syndrome, and olivopontocerebellar atrophy, and Huntington disease; spinocerebellar degenerations, including spinocerebellar ataxias, including Friedreich ataxia, and ataxia-telanglectasia, degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease, mitochondrial encephalomyopathies, including Leigh disease and other mitochondrial encephalomyopathies; toxic and acquired metabolic diseases, including vitamin deficiencies such as thiamine (vitamin B₁) deficiency and vitamin B₁₂ deficiency, neurologic sequelae of metabolic disturbances, including hypoglycemia, hyperglycemia, and hepatic encephatopathy, toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma), and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease.

Disorders involving the colon include, but are not limited to, congenital anomalies, such as atresia and stenosis, Meckel diverticulum, congenital aganglionic megacolon-Hirschsprung disease; enterocolitis, such as diarrhea and dysentery, infectious enterocolitis, including viral gastroenteritis, bacterial enterocolitis, necrotizing enterocolitis, antibiotic-associated colitis (pseudomembranous colitis), and collagenous and lymphocytic colitis, miscellaneous intestinal inflammatory disorders, including parasites and protozoa, acquired immunodeficiency syndrome, transplantation, drug-induced intestinal injury, radiation enterocolitis, neutropenic colitis (typhlitis), and diversion colitis; idiopathic inflammatory bowel disease, such as Crohn's disease and ulcerative colitis; tumors of the colon, such as non-neoplastic polyps, adenomas, familial syndromes, colorectal carcinogenesis, colorectal carcinoma, and carcinoid tumors.

Disorders associated with the liver include, but are not limited to, those arising from an accumulation in the liver of fibrous tissue, such as that resulting from an imbalance between production and degradation of the extracellular matrix accompanied by the collapse and condensation of preexisting fibers; hepatocellular necrosis or injury induced by a wide variety of agents including processes which disturb homeostasis, such as an inflammatory process, tissue damage resulting from toxic injury or altered hepatic blood flow, and infections (e.g., bacterial, viral and parasitic); and portal hypertension or hepatic fibrosis, e.g., fibrosis resulting from a storage disorder such as Gaucher's disease (lipid abnormalities) or a glycogen storage disease, Al-antitrypsin deficiency; a disorder mediating the accumulation (e.g., storage) of an exogenous substance, for example, hemochromatosis (iron-overload syndrome) and copper storage diseases (Wilson's disease), disorders resulting in the accumulation of a toxic metabolite (e.g., tyrosinemia, fructosemia and galactosemia) and peroxisomal disorders (e.g., Zellweger syndrome). Additionally, liver disorders can include injury associated with the administration of various chemicals or drugs, such as for example, methotrexate, isonizaid, oxyphenisatin, methyldopa, chlorpromazine, tolbutamide or alcohol, or a hepatic manifestation of a vascular disorder such as obstruction of either the intrahepatic or extrahepatic bile flow or an alteration in hepatic circulation resulting, for example, from chronic heart failure, veno-occlusive disease, portal vein thrombosis or Budd-Chiari syndrome.

Salivary gland disorders include, but are not limited to sialadenitis, mumps, xerostomia, Mikulicz's syndrome, sialolithiasis, Rosai-Dorfman disease, lacrimo-auriculodentodigital syndrome, hypoplasia/agenesis of the salivary gland, polycystic-dysgenetic disease of the salivary glands and Wegener's granulomatosis.

Furthermore, 69624 may play an important role in the regulation of metabolism. Diseases of metabolic imbalance include, but are not limited to, obesity, anorexia nervosa, cachexia, lipid disorders, and diabetes.

The 69624 protein, fragments thereof, and derivatives and other variants of the sequence in SEQ ID NO:2 thereof are collectively referred to as "polypeptides or proteins of the invention" or "69624 polypeptides or proteins". Nucleic acid molecules encoding such polypeptides or proteins are collectively referred to as "nucleic acids of the invention" or "69624 nucleic acids." 69624 molecules refer to 69624 nucleic acids, polypeptides, and antibodies.

As used herein, the term "nucleic acid molecule" includes DNA molecules (e.g., a cDNA or genomic DNA), RNA molecules (e.g., an mRNA) and analogs of the DNA or RNA. A DNA or RNA analog can be synthesized from nucleotide analogs. The nucleic acid molecule can be single-stranded or double-stranded, but preferably is double-stranded DNA.

The term "isolated nucleic acid molecule" or "purified nucleic acid molecule" includes nucleic acid molecules that are separated from other nucleic acid molecules present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in *Current Protocols in Molecular Biology,* John Wiley & Sons, N.Y. (1989), 6.3.1-6.3.6, which is incorporated by reference. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

Preferably, an isolated nucleic acid molecule of the invention that hybridizes under a stringency condition described herein to the sequence of SEQ ID NO: or SEQ ID NO:3, corresponds to a naturally-occurring nucleic acid molecule.

As used herein, a "naturally-occurring" nucleic acid molecule refers to an RNA or DNA molecule having a nucleotide sequence that occurs in nature. For example a naturally occurring nucleic acid molecule can encode a natural protein.

As used herein, the terms "gene" and "recombinant gene" refer to nucleic acid molecules which include at least an open reading frame encoding a 69624 protein. The gene can optionally further include non-coding sequences, e.g., regulatory sequences and introns. Preferably, a gene encodes a mammalian 69624 protein or derivative thereof

An "isolated" or "purified" polypeptide or protein is substantially free of cellular material or other contaminating proteins from the cell or tissue source from which the protein is derived, or substantially free from chemical precursors or other chemicals when chemically synthesized. "Substantially free" means that a preparation of 69624 protein is at least 10% pure. In a preferred embodiment, the preparation of 69624 protein has less than about 30%, 20%, 10% and more preferably 5% (by dry weight), of non-69624 protein (also referred to herein as a "contaminating protein"), or of chemical precursors or non-69624 chemicals. When the 69624 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 69624 without abolishing or substantially altering a 69624 activity. Preferably the alteration does not substantially alter the 69624 activity, e.g., the activity is at least 20%, 40%, 60%, 70% or 80% of wild-type. An "essential" amino acid residue is a residue that, when altered from the wild-type sequence of 69624, results in abolishing a 69624 activity such that less than 20% of the wild-type activity is present. For example, conserved amino acid residues in 69624 are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a 69624 protein is preferably replaced with another amino acid residue from the same side chain family. Alternatively, in another embodiment, mutations can be introduced randomly along all or part of a 69624 coding sequence, such as by saturation mutagenesis, and the resultant mutants can be screened for 69624 biological activity to identify mutants that retain activity. Following mutagenesis of SEQ ID NO: 1 or SEQ ID NO:3, the encoded protein can be expressed recombinantly and the activity of the protein can be determined.

As used herein, a "biologically active portion" of a 69624 protein includes a fragment of a 69624 protein which participates in an interaction, e.g., an intramolecular or an inter-molecular interaction. An inter-molecular interaction can be a specific binding interaction or an enzymatic interaction (e.g., the interaction can be transient and a covalent bond is formed or broken). An inter-molecular interaction can be between a 69624 molecule and a non-69624 molecule or between a first 69624 molecule and a second 69624 molecule (e.g., a dimerization interaction). Biologically active portions of a 69624 protein include peptides comprising amino acid sequences sufficiently homologous to or derived from the amino acid sequence of the 69624 protein, e.g., the amino acid sequence shown in SEQ ID NO:2, which include less amino acids than the full length 69624 proteins, and exhibit at least one activity of a 69624 protein. Typically, biologically active portions comprise a domain or motif with at least one activity of the 69624 protein, e.g., transport of sodium and dicarboxylate or sulfate ions across biological membranes. A biologically active portion of a 69624 protein can be a polypeptide which is, for example, 10, 25, 50, 100, 150, 200, 250, 300, 350, 400, 450 or more amino acids in length. Biologically active portions of a 69624 protein can be used as targets for developing agents which modulate a 69624 mediated activity, e.g., transport of sodium and dicarboxylate or sulfate ions across biological membranes.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) *J*. *Mol. Biol.* 48:444-453 ) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, *et al.* (1990) *J*. *Mol. Biol.* 215:403-10. BLAST nucleotide searches can be performed with the NBLAST program, score = 100, wordlength = 12 to obtain nucleotide sequences homologous to 69624 nucleic acid molecules of the invention. BLAST protein searches can be performed with the XBLAST program, score = 50, wordlength = 3 to obtain amino acid sequences homologous to 69624 protein molecules of the invention. To obtain gapped alignments for comparison purposes, Gapped BLAST can be utilized as described in Altschul *et al*., (1997) *Nucleic Acids Res.* 25:3389-3402. When utilizing BLAST and Gapped BLAST programs, the default parameters of the respective programs (e.g., XBLAST and NBLAST) can be used. See http://www.ncbi.nlm.nih.gov.

Particular 69624 polypeptides of the present invention have an amino acid sequence substantially identical to the amino acid sequence of SEQ ID NO:2. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 60%, or 65% identity, likely 75% identity, more likely 85%, 90%. 91%, 92%, 93%, 94%, 95%, 96%, 97%, 98% or 99% identity to SEQ ID NO:2 are termed substantially identical.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 60%, or 65% identity, likely 75% identity, more likely 85%, 90%. 91%, 92%, 93%, 94%, 95%, 96%, 97%, 98% or 99% identity to SEQ ID NO: 1 or 3 are termed substantially identical.

"Misexpression or aberrant expression", as used herein, refers to a non-wildtype pattern of gene expression at the RNA or protein level. It includes: expression at non-wild type levels, i.e., over- or under-expression; a pattern of expression that differs from wild type in terms of the time or stage at which the gene is expressed, e.g., increased or decreased expression (as compared with wild type) at a predetermined developmental period or stage; a pattern of expression that differs from wild type in terms of altered, e.g., increased or decreased, expression (as compared with wild type) in a predetermined cell type or tissue type; a pattern of expression that differs from wild type in terms of the splicing size, translated amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus.

"Subject," as used herein, refers to human and non-human animals. The term "non-human animals" of the invention includes all vertebrates, e.g., mammals, such as non-human primates (particularly higher primates), sheep, dog, rodent (e.g., mouse or rat), guinea pig, goat, pig, cat, rabbits, cow, and non-mammals, such as chickens, amphibians, reptiles, etc. In a preferred embodiment, the subject is a human. In another embodiment, the subject is an experimental animal or animal suitable as a disease model.

A "purified preparation of cells", as used herein, refers to an in vitro preparation of cells. In the case cells from multicellular organisms (e.g., plants and animals), a purified preparation of cells is a subset of cells obtained from the organism, not the entire intact organism. In the case of unicellular microorganisms (e.g., cultured cells and microbial cells), it consists of a preparation of at least 10% and more preferably 50% of the subject cells.

Various aspects of the invention are described in further detail below.

### Isolated Nucleic Acid Molecules

In one aspect, the invention provides, an isolated or purified, nucleic acid molecule that encodes a 69624 polypeptide described herein, e.g., a full-length 69624 protein or a fragment thereof, e.g., a biologically active portion of 69624 protein. Also included is a nucleic acid fragment suitable for use as a hybridization probe, which can be used, e.g., to identify a nucleic acid molecule encoding a polypeptide of the invention, 69624 mRNA, and fragments suitable for use as primers, e.g., PCR primers for the amplification or mutation of nucleic acid molecules.

In one embodiment, an isolated nucleic acid molecule of the invention includes the nucleotide sequence shown in SEQ ID NO:1, or a portion of any of these nucleotide sequences. In one embodiment, the nucleic acid molecule includes sequences encoding the human 69624 protein (i.e., "the coding region" of SEQ ID NO:1, as shown in SEQ ID NO:3), as well as 5' untranslated sequences. Alternatively, the nucleic acid molecule can include only the coding region of SEQ ID NO:1 (e.g., SEQ ID NO:3 and, e.g., no flanking sequences which normally accompany the subject sequence. In another embodiment, the nucleic acid molecule encodes a sequence corresponding to a fragment of the protein from about amino acid 6 to 554 of SEQ ID NO:2, or a fragment thereof, e.g. about amino acid residues 6 to 200, 41 to 167, 200 to 400, 202 to 375, 401 to 554 or 498 to 514 of SEQ ID NO:2.

In another embodiment, an isolated nucleic acid molecule of the invention includes a nucleic acid molecule which is a complement of the nucleotide sequence shown in SEQ ID NO:1_or SEQ ID NO:3, or a portion of any of these nucleotide sequences. In other embodiments, the nucleic acid molecule of the invention is sufficiently complementary to the nucleotide sequence shown in SEQ ID NO:1 or SEQ ID NO:3, such that it can hybridize (e.g., under a stringency condition described herein) to the nucleotide sequence shown in SEQ ID NO:1 or 3, thereby forming a stable duplex.

In one embodiment, an isolated nucleic acid molecule of the present invention includes a nucleotide sequence which is at least about: 60%, 65%, 70%, 75%, 80%, 85%, 90%, 91%, 92%, 93%, 94%, 95%, 96%, 97%, 98%, 99%, or more homologous to the entire length of the nucleotide sequence shown in SEQ ID NO:1 or SEQ ID NO:3, or a portion, preferably of the same length, of any of these nucleotide sequences.

### 69624 Nucleic Acid Fragments

A nucleic acid molecule of the invention can include only a portion of the nucleic acid sequence of SEQ ID NO:1 or 3. For example, such a nucleic acid molecule can include a fragment which can be used as a probe or primer or a fragment encoding a portion of a 69624 protein, e.g., an immunogenic or biologically active portion of a 69624 protein. A fragment can comprise those nucleotides of SEQ ID NO:1, which encode a sodium dicarboxylate/sulfate transporter domain of human 69624. The nucleotide sequence determined from the cloning of the 69624 gene allows for the generation of probes and primers designed for use in identifying and/or cloning other 69624 family members, or fragments thereof, as well as 69624 homologues, or fragments thereof, from other species.

In another embodiment, a nucleic acid includes a nucleotide sequence that includes part, or all, of the coding region and extends into either (or both) the 5' or 3' noncoding region. Other embodiments include a fragment which includes a nucleotide sequence encoding an amino acid fragment described herein. Nucleic acid fragments can encode a specific domain or site described herein or fragments thereof, particularly fragments thereof which are at least 100, 150, 200, 250, 300, 350, 400, or 450 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, a 69624 nucleic acid fragment can include a sequence corresponding to a sodium dicarboxylate/sulfate transporter domain or a sodium sulfate symporter signature motif.

69624 probes and primers are provided. Typically a probe/primer is an isolated or purified oligonucleotide. The oligonucleotide typically includes a region of nucleotide sequence that hybridizes under a stringency condition described herein to at least about 7, 12 or 15, preferably about 20 or 25, more preferably about 30, 35, 40, 45, 50, 55, 60, 65, or 75 consecutive nucleotides of a sense or antisense sequence of SEQ ID NO:1 or SEQ ID NO:3, or of a naturally occurring allelic variant or mutant of SEQ ID NO: 1 or SEQ ID NO:3.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a 69624 nucleic acid which encodes a sodium dicarboxylate/sulfate transporter domain, e.g., about nucleotides 27 to 1673 of SEQ ID NO:1, or a sodium sulfate symporter signature sequence, e.g., about nucleotides 1503 to 1553 of SEQ ID NO:1.

In another embodiment a set of primers is provided, e.g., primers suitable for use in a PCR, which can be used to amplify a selected region of a 69624 sequence, e.g., a domain, region, site or other sequence described herein. The primers should be at least 5, 10, or 50 base pairs in length and less than 100, or less than 200, base pairs in length. The primers should be identical, or differs by one base from a sequence disclosed herein or from a naturally occurring variant. For example, primers suitable for amplifying all or a portion of any of the following regions are provided: a nucleotide sequence that encodes a sodium dicarboxylate/sulfate transporter domain, e.g., from about nucleotides 27 to 1673 of SEQ ID NO:1; or a nucleotide sequence that encodes a sodium sulfate symproter signature motif, e.g., from about nucleotides 1503 to 1553 of SEQ ID NO:1.

A nucleic acid fragment can encode an epitope bearing region of a polypeptide described herein. In one embodiment, a nucleic acid fragment encoding an epitope bearing region of a 69624 polypeptide can encode about amino acids 545 to 568 of SEQ ID NO:2 or a fragment thereof e.g. about amino acids 553 to 560 or 561 to 568 of SEQ ID NO:2.

A nucleic acid fragment encoding a "biologically active portion of a 69624 polypeptide" can be prepared by isolating a portion of the nucleotide sequence of SEQ ID NO:1 or 3, which encodes a polypeptide having a 69624 biological activity (e.g., the biological activities of the 69624 proteins are described herein), expressing the encoded portion of the 69624 protein (e.g., by recombinant expression *in vitro)* and assessing the activity of the encoded portion of the 69624 protein. For example, a nucleic acid fragment encoding a biologically active portion of 69624 includes a sodium dicarboxylate/sulfate transporter domain, e.g., amino acid residues about 6 to 554 of SEQ ID NO:2 or a subdomain, e.g. about amino acid residues 41 to 167 or 202 to 375 of SEQ ID NO:2. A nucleic acid fragment encoding a biologically active portion of a 69624 polypeptide, can comprise a nucleotide sequence which is greater than 300, 350, 400, 450 or more nucleotides in length, e.g. about 381 nucleotides, about 522 nucleotides or about 1647 nucleotides in length.

In preferred embodiments, a nucleic acid includes a nucleotide sequence which is about 300, 400, 500, 600, 700, 800, 900, 1000, 1100, 1200, 1300, 1400, 1500, 1600, 1700, 1800, 1900, 2000, 2100, 2200, 2300, 2400, 2500 or more nucleotides in length and hybridizes under a stringency condition described herein to a nucleic acid molecule of SEQ ID NO:1, or SEQ ID NO:3.

### 69624 Nucleic Acid Variants

The invention further encompasses nucleic acid molecules that differ from the nucleotide sequence shown in SEQ ID NO:1 or SEQ ID NO:3. Such differences can be due to degeneracy of the genetic code (and result in a nucleic acid which encodes the same 69624 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:2. If alignment is needed for this comparison the sequences should be aligned for maximum homology. "Looped" out sequences from deletions or insertions, or mismatches, are considered differences.

Nucleic acids of the inventor can be chosen for having codons, which are preferred, or non-preferred, for a particular expression system. E.g., the nucleic acid can be one in which at least one codon, at preferably at least 10%, or 20% of the codons has been altered such that the sequence is optimized for expression in *E. coli,* yeast, human, insect, or CHO cells.

Nucleic acid variants can be naturally occurring, such as allelic variants (same locus), homologs (different locus), and orthologs (different organism) or can be non naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

In a preferred embodiment, the nucleic acid differs from that of SEQ ID NO: 1 or 3, e.g., as follows: by at least one but less than 10, 20, 30, or 40 nucleotides; at least one but less than 1%, 5%, 10% or 20% of the nucleotides in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more identical to the nucleotide sequence shown in SEQ ID NO:2 or a fragment of this sequence. Such nucleic acid molecules can readily be identified as being able to hybridize under a stringency condition described herein, to the nucleotide sequence shown in SEQ ID NO 2 or a fragment of the sequence. Nucleic acid molecules corresponding to orthologs, homologs, and allelic variants of the 69624 cDNAs of the invention can further be isolated by mapping to the same chromosome or locus as the 69624 gene, which was mapped to human chromosome 17.

Preferred variants include those that are correlated with the transport of sodium and dicarboxylate or sulfate ions across a biological membrane.

Allelic variants of 69624, e.g., human 69624, include both functional and non-functional proteins. Functional allelic variants are naturally occurring amino acid sequence variants of the 69624 protein within a population that maintain the ability to transport sodium and dicarboxylate or sulfate ions across a biological membrane.
Functional allelic variants will typically contain only conservative substitution of one or more amino acids of SEQ ID NO:2, or substitution, deletion or insertion of non-critical residues in non-critical regions of the protein. Non-functional allelic variants are naturally-occurring amino acid sequence variants of the 69624, e.g., human 69624, protein within a population that do not have the ability to transport sodium and dicarboxylate or sulfate ions across a biological membrane. Non-functional allelic variants will typically contain a non-conservative substitution, a deletion, or insertion, or premature truncation of the amino acid sequence of SEQ ID NO:2, or a substitution, insertion, or deletion in critical residues or critical regions of the protein.

Moreover, nucleic acid molecules encoding other 69624 family members and, thus, which have a nucleotide sequence which differs from the 69624 sequences of SEQ ID NO:1 or SEQ ID NO:3 are intended to be within the scope of the invention.

### Antisense Nucleic Acid Molecules, Ribozymes and Modified 69624 Nucleic Acid Molecules

In another aspect, the invention features, an isolated nucleic acid molecule which is antisense to 69624. An "antisense" nucleic acid can include a nucleotide sequence which is complementary to a "sense" nucleic acid encoding a protein, e.g., complementary to the coding strand of a double-stranded cDNA molecule or complementary to an mRNA sequence. The antisense nucleic acid can be complementary to an entire 69624 coding strand, or to only a portion thereof (e.g., the coding region of human 69624 corresponding to SEQ ID NO:3). In another embodiment, the antisense nucleic acid molecule is antisense to a "noncoding region" of the coding strand of a nucleotide sequence encoding 69624 (e.g., the 5' and 3' untranslated regions).

An antisense nucleic acid can be designed such that it is complementary to the entire coding region of 69624 mRNA, but more preferably is an oligonucleotide which is antisense to only a portion of the coding or noncoding region of 69624 mRNA. For example, the antisense oligonucleotide can be complementary to the region surrounding the translation start site of 69624 mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject (e.g., by direct injection at a tissue site), or generated *in situ* such that they hybridize with or bind to cellular mRNA and/or genomic DNA encoding a 69624 protein to thereby inhibit expression of the protein, e.g., by inhibiting transcription and/or translation. Alternatively, antisense nucleic acid molecules can be modified to target selected cells and then administered systemically. For systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al.* (1987) *Nucleic Acids. Res.* 15:6625-6641). The antisense nucleic acid molecule can also comprise a 2'-o-methylribonucleotide (Inoue *et al.* (1987) *Nucleic Acids Res.* 15:6131-6148) or a chimeric RNA-DNA analogue (Inoue *et al.* (1987) *FEES Lett.* 215:327-330).

In still another embodiment, an antisense nucleic acid of the invention is a ribozyme. A ribozyme having specificity for a 69624-encoding nucleic acid can include one or more sequences complementary to the nucleotide sequence of a 69624 cDNA disclosed herein (i.e., SEQ ID NO:1 or SEQ ID NO:3), and a sequence having known catalytic sequence responsible for mRNA cleavage (see U.S. Pat. No. 5,093,246 or Haselhoff and Gerlach (1988) *Nature* 334:585-591). For example, a derivative of a *Tetrahymena* L-19 IVS RNA can be constructed in which the nucleotide sequence of the active site is complementary to the nucleotide sequence to be cleaved in a 69624-encoding mRNA. See, e.g., Cech *et al.* U.S. Patent No. 4,987,071; and Cech *et al.* U.S. Patent No. 5,116,742. Alternatively, 69624 mRNA can be used to select a catalytic RNA having a specific ribonuclease activity from a pool of RNA molecules. See, e.g., Bartel, D. and Szostak, J.W. (1993) *Science* 261:1411-1418.

69624 gene expression can be inhibited by targeting nucleotide sequences complementary to the regulatory region of the 69624 (e.g., the 69624 promoter and/or enhancers) to form triple helical structures that prevent transcription of the 69624 gene in target cells. See generally, Helene, C. (1991) *Anticancer Drug Des.* 6:569-84; Helene, C. i (1992) *Ann. N.Y*. *Acad. Sci.* 660:27-36; and Maher, L.J. (1992) *Bioassays* 14:807-15. The potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

A 69624 nucleic acid molecule can be modified at the base moiety, sugar moiety or phosphate backbone to improve, e.g., the stability, hybridization, or solubility of the molecule. For non-limiting examples of synthetic oligonucleotides with modifications see Toulmé (2001) *Nature Biotech.* 19:17 and Faria *et al.* (2001) *Nature Biotech.* 19:40-44. Such phosphoramidite oligonucleotides can be effective antisense agents.

For example, the deoxyribose phosphate backbone of the nucleic acid molecules can be modified to generate peptide nucleic acids (see Hyrup B. *et al.* (1996) *Bioorganic & Medicinal Chemistry* 4: 5-23). As used herein, the terms "peptide nucleic acid" or "PNA" refers to a nucleic acid mimic, e.g., a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup B. *et al.* (1996) *supra* and Perry-O'Keefe *et al. Proc. Natl. Acad. Sci.* 93: 14670-675.

PNAs of 69624 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 69624 nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup B. *et al.* (1996) *supra*)); or as probes or primers for DNA sequencing or hybridization (Hyrup B. *et al.* (1996) *supra*; Perry-O'Keefe *supra*).

In other embodiments, the oligonucleotide may include other appended groups such as peptides (e.g., for targeting host cell receptors *in vivo*), or agents facilitating transport across the cell membrane (see, e.g., Letsinger *et al.* (1989) *Proc. Natl. Acad. Sci. USA* 86:6553-6556; Lemaitre *et al.* (1987) *Proc. Natl. Acad. Sci. USA* 84:648-652; PCT Publication No. W088/09810) or the blood-brain barrier (see, e.g., PCT Publication No. W089/10134). In addition, oligonucleotides can be modified with hybridization-triggered cleavage agents (see, e.g., Krol *et al.* (1988) *Bio-Techniques* 6:958-976) or intercalating agents. (see, e.g., Zon (1988) *Pharm. Res.* 5:539-549). To this end, the oligonucleotide may be conjugated to another molecule, (e.g., a peptide, hybridization triggered crosslinking agent, transport agent, or hybridization-triggered cleavage agent).

The invention also includes molecular beacon oligonucleotide primer and probe molecules having at least one region which is complementary to a 69624 nucleic acid of the invention, two complementary regions one having a fluorophore and one a quencher such that the molecular beacon is useful for quantitating the presence of the 69624 nucleic acid of the invention in a sample. Molecular beacon nucleic acids are described, for example, in Lizardi *et al*., U.S. Patent No.5,854,033; Nazarenko *et al*., U.S. Patent No. 5,866,336, and Livak *et al*., U.S. Patent 5,876,930.

### Isolated 69624 Polypeptides

In another aspect, the invention features, an isolated 69624 protein, or fragment, e.g., a biologically active portion, for use as immunogens or antigens to raise or test (or more generally to bind) anti-69624 antibodies. 69624 protein can be isolated from cells or tissue sources using standard protein purification techniques. 69624 protein or fragments thereof can be produced by recombinant DNA techniques or synthesized chemically.

Polypeptides of the invention include those which arise as a result of the existence of multiple genes, alternative transcription events, alternative RNA splicing events, and alternative translational and post-translational events. The polypeptide can be expressed in systems, e.g., cultured cells, which result in substantially the same post-translational modifications present when expressed the polypeptide is expressed in a native cell, or in systems which result in the alteration or omission of post-translational modifications, e.g., glycosylation or cleavage, present when expressed in a native cell.

In a preferred embodiment, a 69624 polypeptide has one or more of the following characteristics:
it has the ability to transport sodium ions across a biological membrane;
(ii) it has the ability to actively transport dicarboxylate or sulfate ions in conjunction with the transport of the sodium ions;
(iii) it has a molecular weight, e.g., a deduced molecular weight, preferably ignoring any contribution of post translational modifications, amino acid composition or other physical characteristic of SEQ ID NO:2;
(iv) it has an overall sequence similarity of at least 60%, preferably at least 70%, more preferably at least 80, 85, 90, or 95%, with a polypeptide a of SEQ ID NO:2;
(v) it can be found in the kidney, brain, colon, liver, or salivary gland;
(vi) it has a sodium dicarboxylate/sulfate transporter domain which is preferably about 70%, 80%, 90% or 95% homologous with amino acid residues about 6 to 554 of SEQ ID NO:2;
(vii) it has a sodium sulfate symporter signature motif (PS01271);
(viii) it has at least one, two, three, four, five, six, seven, eight, nine, ten, and preferably eleven, twelve, or thirteen transmembrane domains;
(ix) it has at least one, preferably two predicted Protein kinase C phosphorylation sites (PS00005);
(x) it has at least one, preferably two predicted Casein kinase II phosphorylation sites (PS00006);
(xi) it has at least one, two, preferably three predicted N-myristoylation sites (PS00008); and
(xi) it has at least one, two, preferably three predicted N-myristoylation sites (PS00008); and
(xii) it has at least one predicted N-glycosylation site (PS00001).

In a preferred embodiment the 69624 protein, or fragment thereof, differs from the corresponding sequence in SEQ ID:2. In one embodiment it differs by at least one but by less than 15, 10 or 5 amino acid residues. In another it differs from the corresponding sequence in SEQ ID NO:2 by at least one residue but less than 20%, 15%, 10% or 5% of the residues in it differ from the corresponding sequence in SEQ ID NO:2. (If this comparison requires alignment the sequences should be aligned for maximum homology. "Looped" out sequences from deletions or insertions, or mismatches, are considered differences.) The differences are, preferably, differences or changes at a non essential residue or a conservative substitution. In a preferred embodiment the differences are not in the sodium dicarboxylate/sulfate transporter domain, e.g., about amino acid residues 6 to 554 of SEQ ID NO:2. In another preferred embodiment one or more differences are in the sodium dicarboxylate/sulfate transporter domain, e.g., about amino acid residues 6 to 554 of SEQ ID NO:2.

Other embodiments include a protein that contains one or more changes in amino acid sequence, e.g., a change in an amino acid residue which is not essential for activity. Such 69624 proteins differ in amino acid sequence from SEQ ID NO:2, yet retain biological activity.

In one embodiment, the protein includes an amino acid sequence at least about 60%, 65%, 70%, 75%, 80%, 85%, 90%, 95%, 96%, 97%, 98%, 99% or more homologous to SEQ ID NO:2. In another embodiment, the protein includes fragments or regions homologous to fragments, at least about 70%, 80%, 90%, 95%, 96%, 97%, 98%, 99% or more homologous to a fragment of SEQ ID NO:2. A fragment of a 69624 protein can be a domain, e.g. a sodium dicarboxylate/sulfate transporter domain, e.g., about amino acid residues 6 to 554 of SEQ ID NO:2 or a fragment thereof, e.g. about amino acid residues 6 to 200, 41 to 167, 200 to 400, 202 to 375, 401 to 554 or 498 to 514 of SEQ ID NO:2.

A 69624 protein or fragment is provided which varies from the sequence of SEQ ID NO:2 in regions defined by amino acid residues about 141 to 209, 273 to 308, 371 to 405, and 555 to 568 by at least one but by less than 15, 10 or 5 amino acid residues in the protein or fragment, but which does not differ from SEQ ID NO:2 in regions defined by amino acid residues about 1 to 140, 210 to 272, 309 to 370, and 406 to 554. Alternatively, a 69624 protein or fragment is provided which varies form the sequence of SEQ ID NO:2 in regions defined by amino acid residues about 371 to 568 by at least one but by less than 15, 10, or 5 amino acid residues in the protein or fragment, but which does not differ from SEQ ID NO:2 in regions defined by amino acid residues 1 to 370. (If these comparisons require alignment the sequences should be aligned for maximum homology. "Looped" out sequences from deletions or insertions, or mismatches, are considered differences.) In some embodiments the difference is at a non-essential residue or is a conservative substitution, while in others the difference is at an essential residue or is a non-conservative substitution.

In one embodiment, a biologically active portion of a 69624 protein includes a sodium dicarboxylate/sulfate transporter domain. Moreover, other biologically active portions, in which other regions of the protein are deleted, can be prepared by recombinant techniques and evaluated for one or more of the functional activities of a native 69624 protein.

In a preferred embodiment, the 69624 protein has an amino acid sequence shown in SEQ ID NO:2. In other embodiments, the 69624 protein is substantially identical to SEQ ID NO:2. In yet another embodiment, the 69624 protein is substantially identical to SEQ ID NO:2 and retains the functional activity of the protein of SEQ ID NO:2, as described in detail in the subsections above.

### 69624 Chimeric or Fusion Proteins

In another aspect, the invention provides 69624 chimeric or fusion proteins. As used herein, a 69624 "chimeric protein" or "fusion protein" includes a 69624 polypeptide linked to a non-69624 polypeptide. A "non-69624 polypeptide" refers to a polypeptide having an amino acid sequence corresponding to a protein which is not substantially homologous to the 69624 protein, e.g., a protein which is different from the 69624 protein and which is derived from the same or a different organism. The 69624 polypeptide of the fusion protein can correspond to all or a portion e.g., a fragment described herein of a 69624 amino acid sequence. In a preferred embodiment, a 69624 fusion protein includes at least one (or two) biologically active portion of a 69624 protein. The non-69624 polypeptide can be fused to the N-terminus or C-terminus of the 69624 polypeptide.

The fusion protein can include a moiety which has a high affinity for a ligand. For example, the fusion protein can be a GST-69624 fusion protein in which the 69624 sequences are fused to the C-terminus of the GST sequences. Such fusion proteins can facilitate the purification of recombinant 69624. Alternatively, the fusion protein can be a 69624 protein containing a heterologous signal sequence at its N-terminus. In certain host cells (e.g., mammalian host cells), expression and/or secretion of 69624 can be increased through use of a heterologous signal sequence.

Fusion proteins can include all or a part of a serum protein, e.g., an IgG constant region, or human serum albumin.

The 69624 fusion proteins of the invention can be incorporated into pharmaceutical compositions and administered to a subject *in vivo.* The 69624 fusion proteins can be used to affect the bioavailability of a 69624 substrate. 69624 fusion proteins may be useful therapeutically for the treatment of disorders caused by, for example, (i) aberrant modification or mutation of a gene encoding a 69624 protein; (ii) mis-regulation of the 69624 gene; and (iii) aberrant post-translational modification of a 69624 protein.

Moreover, the 69624-fusion proteins of the invention can be used as immunogens to produce anti-69624 antibodies in a subject, to purify 69624 ligands and in screening assays to identify molecules which inhibit the interaction of 69624 with a 69624 substrate.

Expression vectors are commercially available that already encode a fusion moiety (e.g., a GST polypeptide). A 69624-encoding nucleic acid can be cloned into such an expression vector such that the fusion moiety is linked in-frame to the 69624 protein.

### Variants of 69624 Proteins

In another aspect, the invention also features a variant of a 69624 polypeptide, e.g., which functions as an agonist (mimetics) or as an antagonist. Variants of the 69624 proteins can be generated by mutagenesis, e.g., discrete point mutation, the insertion or deletion of sequences or the truncation of a 69624 protein. An agonist of the 69624 proteins can retain substantially the same, or a subset, of the biological activities of the naturally occurring form of a 69624 protein. An antagonist of a 69624 protein can inhibit one or more of the activities of the naturally occurring form of the 69624 protein by, for example, competitively modulating a 69624-mediated activity of a 69624 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 69624 protein.

Variants of a 69624 protein can be identified by screening combinatorial libraries of mutants, e.g., truncation mutants, of a 69624 protein for agonist or antagonist activity.

Libraries of fragments e.g., N terminal, C terminal, or internal fragments, of a 69624 protein coding sequence can be used to generate a variegated population of fragments for screening and subsequent selection of variants of a 69624 protein. Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of 69624 proteins. Recursive ensemble mutagenesis (REM), a new technique which enhances the frequency of functional mutants in the libraries, can be used in combination with the screening assays to identify 69624 variants (Arkin and Yourvan (1992) *Proc. Natl. Acad. Sci. USA* 89:7811-7815; Delgrave *et al.* (1993) *Protein Engineering* 6:327-331).

Cell based assays can be exploited to analyze a variegated 69624 library. For example, a library of expression vectors can be transfected into a cell line, e.g., a cell line, which ordinarily responds to 69624 by transporting an appropriate substrate, e.g., citrate, succinate, or sufate, into the cell in a sodium-dependent manner. The transfected cells are then contacted with 69624 and the effect of the expression of the mutant on transport of the 69624 substrate can be detected, e.g., by measuring uptake of a radiolabeled substrate in the presence or absence of sodium ions. Plasmid DNA can then be recovered from the cells which score for inhibition, or alternatively, potentiation of signaling by the 69624 substrate, and the individual clones further characterized.

In another aspect, the invention features a method of making a 69624 polypeptide, e.g., a peptide having a non-wild type activity, e.g., an antagonist, agonist, or super agonist of a naturally occurring 69624 polypeptide, e.g., a naturally occurring 69624 polypeptide. The method includes: altering the sequence of a 69624 polypeptide, e.g., altering the sequence , e.g., by substitution or deletion of one or more residues of a non-conserved region, a domain or residue disclosed herein, and testing the altered polypeptide for the desired activity.

In another aspect, the invention features a method of making a fragment or analog of a 69624 polypeptide a biological activity of a naturally occurring 69624 polypeptide. The method includes: altering the sequence, e.g., by substitution or deletion of one or more residues, of a 69624 polypeptide, e.g., altering the sequence of a non-conserved region, or a domain or residue described herein, and testing the altered polypeptide for the desired activity.

### Anti-69624 Antibodies

In another aspect, the invention provides an anti-69624 antibody, or a fragment thereof (e.g., an antigen-binding fragment thereof). The term "antibody" as used herein refers to an immunoglobulin molecule or immunologically active portion thereof, i.e., an antigen-binding portion. As used herein, the term "antibody" refers to a protein comprising at least one, and preferably two, heavy (H) chain variable regions (abbreviated herein as VH), and at least one and preferably two light (L) chain variable regions (abbreviated herein as VL). The VH and VL regions can be further subdivided into regions of hypervariability, termed "complementarity determining regions" ("CDR"), interspersed with regions that are more conserved, termed "framework regions" (FR). The extent of the framework region and CDR's has been precisely defined (see, Kabat, E.A., *et al.* (1991)

*Sequences of Proteins of Immunological Interest*, *Fifth Edition,* U.S. Department of Health and Human Services, NIH Publication No. 91-3242, and Chothia, C. et al. (1987) J. Mol. Biol. 196:901-917, which are incorporated herein by reference). Each VH and VL is composed of three CDR's and four FRs, arranged from amino-terminus to carboxy-terminus in the following order: FR1, CDR1, FR2, CDR2, FR3, CDR3, FR4.

The anti-69624 antibody can further include a heavy and light chain constant region, to thereby form a heavy and light immunoglobulin chain, respectively. In one embodiment, the antibody is a tetramer of two heavy immunoglobulin chains and two light immunoglobulin chains, wherein the heavy and light immunoglobulin chains are interconnected by, e.g., disulfide bonds. The heavy chain constant region is comprised of three domains, CH1, CH2 and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system.

As used herein, the term "immunoglobulin" refers to a protein consisting of one or more polypeptides substantially encoded by immunoglobulin genes. The recognized human immunoglobulin genes include the kappa, lambda, alpha (IgAl and IgA2), gamma (IgG1, IgG2, IgG3, IgG4), delta, epsilon and mu constant region genes, as well as the myriad immunoglobulin variable region genes. Full-length immunoglobulin "light chains" (about 25 KDa or 214 amino acids) are encoded by a variable region gene at the NH2-terminus (about 110 amino acids) and a kappa or lambda constant region gene at the

COOH--terminus. Full-length immunoglobulin "heavy chains" (about 50 KDa or 446

amino acids), are similarly encoded by a variable region gene (about 116 amino acids) and one of the other aforementioned constant region genes, e.g., gamma (encoding about 330 amino acids).

The term "antigen-binding fragment" of an antibody (or simply "antibody portion," or "fragment"), as used herein, refers to one or more fragments of a full-length antibody that retain the ability to specifically bind to the antigen, e.g., 69624 polypeptide or fragment thereof. Examples of antigen-binding fragments of the anti-69624 antibody include, but are not limited to: (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward *et al.,* (1989) *Nature* 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g*., Bird *et al.* (1988) *Science* 242:423-426; and Huston *et al.* (1988) *Proc. Natl. Acad. Sci. USA* 85:5879-5883). Such single chain antibodies are also encompassed within the term "antigen-binding fragment" of an antibody. These antibody fragments are obtained using conventional techniques known to those with skill in the art, and the fragments are screened for utility in the same manner as are intact antibodies.

The anti-69624 antibody can be a polyclonal or a monoclonal antibody. In other embodiments, the antibody can be recombinantly produced, e.g., produced by phage display or by combinatorial methods.

Phage display and combinatorial methods for generating anti-69624 antibodies are known in the art (as described in, e.g., Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) *Bio*/*Technology* 9:1370-1372; Hay et al. (1992) *Hum Antibod Hybridomas* 3:81-85; Huse et al. (1989) *Science* 246:1275-1281; Griffths et al. (1993) *EMBO J* 12:725-734; Hawkins et al. (1992) *J Mol Biol* 226:889-896; Clackson et al. (1991) *Nature* 352:624-628; Gram et al. (1992) *PNAS* 89:3576-3580; Garrad et al. (1991) *Bio*/*Technology* 9:1373-1377; Hoogenboom et al. (1991) *Nuc Acid Res* 19:4133-4137; and Barbas et al. (1991) *PNAS* 88:7978-7982, the contents of all of which are incorporated by reference herein).

In one embodiment, the anti-69624 antibody is a fully human antibody (e.g., an antibody made in a mouse which has been genetically engineered to produce an antibody from a human immunoglobulin sequence), or a non-human antibody, e.g., a rodent (mouse or rat), goat, primate (e.g., monkey), camel antibody. Preferably, the non-human antibody is a rodent (mouse or rat antibody). Method of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, e.g., Wood et al. International Application WO 91/00906, Kucherlapati et al. PCT publication WO 91/10741; Lonberg et al. International Application WO 92/03918; Kay et al. International Application 92/03917; Lonberg, N. et al. 1994 *Nature* 368:856-859; Green, L.L. et al. 1994 *Nature Genet.* 7:13-21; Morrison, S.L. et al. 1994 *Proc. Natl. Acad. Sci. USA* 81:6851-6855; Bruggeman et al. 1993 *Year Immunol* 7:33-40; Tuaillon et al. 1993 *PNAS* 90:3720-3724; Bruggeman et al. 1991 *Eur J Immunol* 21:1323-1326).

An anti-69624 antibody can be one in which the variable region, or a portion thereof, e.g., the CDR's, are generated in a non-human organism, e.g., a rat or mouse. Chimeric, CDR-grafted, and humanized antibodies are within the invention. Antibodies generated in a non-human organism, e.g., a rat or mouse, and then modified, e.g., in the variable framework or constant region, to decrease antigenicity in a human are within the invention.

Chimeric antibodies can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted (see Robinson et al., International Patent Publication PCT/US86/02269; Akira, et al., European Patent Application 184,187; Taniguchi, M., European Patent Application 171,496; Morrison et al., European Patent Application 173,494; Neuberger et al., International Application WO 86/01533; Cabilly et al. U.S. Patent No. 4,816,567; Cabilly et al., European Patent Application 125,023; Better et al. (1988 *Science* 240:1041-1043); Liu et al. (1987) *PNAS* 84:3439-3443; Liu et al., 1987, *J*. *Immunol*. 139:3521-3526; Sun et al. (1987) *PNAS* 84:214-218; Nishimura et al., 1987, *Canc. Res.* 47:999-1005; Wood et al. (1985) *Nature* 314:446-449; and Shaw et al., 1988, *J*. *Natl Cancer Inst.* 80:1553-1559).

A humanized or CDR-grafted antibody will have at least one or two but generally all three recipient CDR's (of heavy and or light immuoglobulin chains) replaced with a donor CDR. The antibody may be replaced with at least a portion of a non-human CDR or only some of the CDR's may be replaced with non-human CDR's. It is only necessary to replace the number of CDR's required for binding of the humanized antibody to a 69624 or a fragment thereof. Preferably, the donor will be a rodent antibody, e.g., a rat or mouse antibody, and the recipient will be a human framework or a human consensus framework. Typically, the immunoglobulin providing the CDR's is called the "donor" and the immunoglobulin providing the framework is called the "acceptor." In one embodiment, the donor immunoglobulin is a non-human (e.g., rodent). The acceptor framework is a naturally-occurring (e.g., a human) framework or a consensus framework, or a sequence about 85% or higher, preferably 90%, 95%, 99% or higher identical thereto.

As used herein, the term "consensus sequence" refers to the sequence formed from the most frequently occurring amino acids (or nucleotides) in a family of related sequences (See e.g., Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

An antibody can be humanized by methods known in the art. Humanized antibodies can be generated by replacing sequences of the Fv variable region which are not directly involved in antigen binding with equivalent sequences from human Fv variable regions. General methods for generating humanized antibodies are provided by Morrison, S. L., 1985, *Science* 229:1202-1207, by Oi et al., 1986, *BioTechniques* 4:214, and by Queen et al. US 5,585,089, US 5,693,761 and US 5,693,762, the contents of all of which are hereby incorporated by reference. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against a 69624 polypeptide or fragment thereof. The recombinant DNA encoding the humanized antibody, or fragment thereof, can then be cloned into an appropriate expression vector.

Humanized or CDR-grafted antibodies can be produced by CDR-grafting or CDR substitution, wherein one, two, or all CDR's of an immunoglobulin chain can be replaced. See e.g., U.S. Patent 5,225,539; Jones et al. 1986 *Nature* 321:552-525; Verhoeyan et al. 1988 *Science* 239:1534; Beidler et al. 1988 *J*. *Immunol.* 141:4053-4060; Winter US 5,225,539, the contents of all of which are hereby expressly incorporated by reference. Winter describes a CDR-grafting method which may be used to prepare the humanized antibodies of the present invention (UK Patent Application GB 2188638A, filed on March 26, 1987; Winter US 5,225,539), the contents of which is expressly incorporated by reference.

Also within the scope of the invention are humanized antibodies in which specific amino acids have been substituted, deleted or added. Preferred humanized antibodies have amino acid substitutions in the framework region, such as to improve binding to the antigen. For example, a humanized antibody will have framework residues identical to the donor framework residue or to another amino acid other than the recipient framework residue. To generate such antibodies, a selected, small number of acceptor framework residues of the humanized immunoglobulin chain can be replaced by the corresponding donor amino acids. Preferred locations of the substitutions include amino acid residues adjacent to the CDR, or which are capable of interacting with a CDR (see e.g., US 5,585,089). Criteria for selecting amino acids from the donor are described in US 5,585,089, e.g., columns 12-16 of US 5,585,089, the e.g., columns 12-16 of US 5,585,089, the contents of which are hereby incorporated by reference. Other techniques for humanizing antibodies are described in Padlan et al. EP 519596 A1, published on December 23, 1992.

In preferred embodiments an antibody can be made by immunizing with purified 69624 antigen, or a fragment thereof, e.g., a fragment described herein, membrane associated antigen, tissue, e.g., crude tissue preparations, whole cells, preferably living cells, lysed cells, or cell fractions, e.g., membrane fractions.

A full-length 69624 protein or antigenic peptide fragment of 69624 can be used as an immunogen or can be used to identify anti-69624 antibodies made with other immunogens, e.g., cells, membrane preparations, and the like. The antigenic peptide or fragment, e.g epitope bearing fragment, of 69624 should include at least 8 amino acid residues of the amino acid sequence shown in SEQ ID NO:2 and encompasses an epitope of 69624. Preferably, the antigenic peptide or fragment includes at least 10 amino acid residues, more preferably at least 15 amino acid residues, even more preferably at least 20 amino acid residues, and most preferably at least 30 amino acid residues.

Fragments of 69624 can be used, for example, to make antibodies or characterize the specificity of an antibody. Fragments of 69624 which include residues 189 to 206, 290 to 308, and 372 to 388 of SEQ ID NO:2 can be used to make, e.g., antibodies against hydrophilic regions of the 69624 protein (see Figure 1). Similarly, fragments of 69624 which include residues about 14 to 31, about 253 to 272, or about 309 to 331 of SEQ ID NO:2 can be used to make an antibody against a hydrophobic region of the 69624 protein; fragments of 69624 which include residues about 141 to 209, about 273 to 308, about 371 to 405, or about 545 to 568 of SEQ ID NO:2 or fragments thereof, e.g. about residues 161 to 181, 553 to 560 or 561 to 568 of SEQ ID NO:2 can be used to make an antibody against a non-transmembrane region of the 69624 protein; and fragments of 69624 which include residues about 423 to 439, about 460 to 489, or about 498 to 554 of SEQ ID NO:2 can be used to make an antibody against the sodium dicarboxylate/sulfate transporter domain of the 69624 protein.

Antibodies reactive with, or specific for, any of these regions, or other regions or domains described herein are provided.

Antibodies which bind only native 69624 protein, only denatured or otherwise non-native 69624 protein, or which bind both, are within the invention. Antibodies with linear or conformational epitopes are within the invention. Conformational epitopes can sometimes be identified by identifying antibodies which bind to native but not denatured 69624 protein.

Preferred epitopes encompassed by the antigenic peptide are regions of 69624 are located on the surface of the protein, e.g., hydrophilic regions, as well as regions with high antigenicity. For example, an Emini surface probability analysis of the human 69624 protein sequence can be used to indicate the regions that have a particularly high probability of being localized to the surface of the 69624 protein and are thus likely to constitute surface residues useful for targeting antibody production.

In a preferred embodiment the antibody can bind to the extracellular portion of the 69624 protein, e.g., it can bind to a whole cell which expresses the 69624 protein. In another embodiment, the antibody binds an intracellular portion of the 69624 protein. In preferred embodiments antibodies can bind one or more of purified antigen, membrane associated antigen, tissue, e.g., tissue sections, whole cells, preferably living cells, lysed cells, cell fractions, e.g., membrane fractions.

The anti-69624 antibody can be a single chain antibody. A single-chain antibody (scFV) may be engineered (see, for example, Colcher, D. *et al.* (1999) *Ann N Y Acad Sci* 880:263-80; and Reiter, Y. (1996) *Clin Cancer Res* 2:245-52). The single chain antibody can be dimerized or multimerized to generate multivalent antibodies having specificities for different epitopes of the same target 69624 protein.

In a preferred embodiment the antibody has: effector function; and can fix complement. In other embodiments the antibody does not; recruit effector cells; or fix complement.

In a preferred embodiment, the antibody has reduced or no ability to bind an Fc receptor. For example, it is an isotype or subtype, fragment or other mutant, which does not support binding to an Fc receptor, e.g., it has a mutagenized or deleted Fc receptor binding region.

In a preferred embodiment, an anti-69624 antibody alters (e.g., increases or decreases) the transport activity of a 69624 polypeptide. For example, the antibody can bind at or in proximity to the active site, e.g., to an epitope that includes a residue located from about 498 to 514 of SEQ ID NO:2.

The antibody can be coupled to a toxin, e.g., a polypeptide toxin, e,g, ricin or diphtheria toxin or active fragment hereof or other therapeutic agent, as described below, or a radioactive nucleus, or imaging agent, e.g. a radioactive, enzymatic, or other, e.g., imaging agent, e.g., a NMR contrast agent. Labels which produce detectable radioactive emissions or fluorescence are preferred.

An anti-69624 antibody (e.g., monoclonal antibody) can be used to isolate 69624 by standard techniques, such as affinity chromatography or immunoprecipitation. Moreover, an anti-69624 antibody can be used to detect 69624 protein (e.g., in a cellular lysate or cell supernatant) in order to evaluate the abundance and pattern of expression of the protein. Anti-69624 antibodies can be used diagnostically to monitor protein levels in tissue as part of a clinical testing procedure, e.g., to determine the efficacy of a given treatment regimen. Detection can be facilitated by coupling (i.e., physically linking) the antibody to a detectable substance (i.e., antibody labelling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

The invention also includes a nucleic acid which encodes an anti-69624 antibody, e.g., an anti-69624 antibody described herein. Also included are vectors which include the nucleic acid and cells transformed with the nucleic acid, particularly cells which are useful for producing an antibody, e.g., mammalian cells, e.g. CHO or lymphatic cells.

The invention also includes cell lines, e.g., hybridomas, which make an anti-69624 antibody, e.g., and antibody described herein, and method of using said cells to make a 69624 antibody.

### Recombinant Expression Vectors, Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

A vector can include a 69624 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (e.g., 69624 proteins, mutant forms of 69624 proteins, fusion proteins, and the like).

The recombinant expression vectors of the invention can be designed for expression of 69624 proteins in prokaryotic or eukaryotic cells. For example, polypeptides of the invention can be expressed in *E*. *coli,* insect cells (e.g., using baculovirus expression vectors), yeast cells or mammalian cells. Suitable host cells are discussed further in Goeddel, (1990) *Gene Expression Technology: Methods in Enzymology* 185, Academic Press, San Diego, CA. Alternatively, the recombinant expression vector can be transcribed and translated *in vitro,* for example using T7 promoter regulatory sequences and T7 polymerase.

Expression of proteins in prokaryotes is most often carried out in E. *coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 69624 activity assays, (e.g., direct assays or competitive assays described in detail below), or to generate antibodies specific for 69624 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (e.g., six weeks).

To maximize recombinant protein expression in *E. coli* is to express the protein in a host bacteria with an impaired capacity to proteolytically cleave the recombinant protein (Gottesman, S., (1990) *Gene Expression Technology: Methods in Enzymology* 185, Academic Press, San Diego, California 119-128). Another strategy is to alter the nucleic acid sequence of the nucleic acid to be inserted into an expression vector so that the individual codons for each amino acid are those preferentially utilized in *E. coli* (Wada *et al*., (1992) *Nucleic Acids Res.* 20:2111-2118). Such alteration of nucleic acid sequences of the invention can be carried out by standard DNA synthesis techniques.

The 69624 expression vector can be a yeast expression vector, a vector for expression in insect cells, e.g., a baculovirus expression vector or a vector suitable for expression in mammalian cells.

When used in mammalian cells, the expression vector's control functions can be provided by viral regulatory elements. For example, commonly used promoters are derived from polyoma, Adenovirus 2, cytomegalovirus and Simian Virus 40.

In another embodiment, the promoter is an inducible promoter, e.g., a promoter regulated by a steroid hormone, by a polypeptide hormone (e.g., by means of a signal transduction pathway), or by a heterologous polypeptide (e.g., the tetracycline-inducible systems, "Tet-On" and "Tet-Off"; see, e.g., Clontech Inc., CA, Gossen and Bujard (1992) *Proc. Natl. Acad. Sci. USA* 89:5547, and Paillard (1989) *Human Gene Therapy* 9:983).

In another embodiment, the recombinant mammalian expression vector is capable of directing expression of the nucleic acid preferentially in a particular cell type (e.g., tissue-specific regulatory elements are used to express the nucleic acid). Non-limiting examples of suitable tissue-specific promoters include the albumin promoter (liver-specific; Pinkert *et al.* (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J* 8:729-733) and immunoglobulins (Banerji *et al.* (1983) *Cell* 33:729-740; Queen and Baltimore (1983) *Cell* 33:741-748), neuron-specific promoters (e.g., the neurofilament promoter; Byrne and Ruddle (1989) *Proc. Natl. Acad. Sci. USA* 86:5473-5477), pancreas-specific promoters (Edlund *et al.* (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example, the murine hox promoters (Kessel and Gruss (1990) *Science* 249:374-379) and the α-fetoprotein promoter (Campes and Tilghman (1989) *Genes Dev.* 3:537-546).

The invention further provides a recombinant expression vector comprising a DNA molecule of the invention cloned into the expression vector in an antisense orientation. Regulatory sequences (e.g., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus.

Another aspect the invention provides a host cell which includes a nucleic acid molecule described herein, e.g., a 69624 nucleic acid molecule within a recombinant expression vector or a 69624 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a 69624 protein can be expressed in bacterial cells (such as E. *coli),* insect cells, yeast or mammalian cells (such as Chinese hamster ovary cells (CHO) or COS cells e.g., COS-7 cells, CV-1 origin SV40 cells; Gluzman (1981) *CellI*23:175-182). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation.

A host cell of the invention can be used to produce (i.e., express) a 69624 protein. Accordingly, the invention further provides methods for producing a 69624 protein using the host cells of the invention. In one embodiment, the method includes culturing the host cell of the invention (into which a recombinant expression vector encoding a 69624 protein has been introduced) in a suitable medium such that a 69624 protein is produced. In another embodiment, the method further includes isolating a 69624 protein from the medium or the host cell.

In another aspect, the invention features, a cell or purified preparation of cells which include a 69624 transgene, or which otherwise misexpress 69624. The cell preparation can consist of human or non-human cells, e.g., rodent cells, e.g., mouse or rat cells, rabbit cells, or pig cells. In preferred embodiments, the cell or cells include a 69624 transgene, e.g., a heterologous form of a 69624, e.g., a gene derived from humans (in the case of a non-human cell). The 69624 transgene can be misexpressed, e.g., overexpressed or underexpressed. In other preferred embodiments, the cell or cells include a gene that mis-expresses an endogenous 69624, e.g., a gene the expression of which is disrupted, e.g., a knockout. Such cells can serve as a model for studying disorders that are related to mutated or mis-expressed 69624 alleles or for use in drug screening.

In another aspect, the invention features, a human cell, e.g., a hepatic stem cell, transformed with nucleic acid which encodes a subject 69624 polypeptide.

Also provided are cells, preferably human cells, e.g., human hepatic or fibroblast cells, in which an endogenous 69624 is under the control of a regulatory sequence that does not normally control the expression of the endogenous 69624 gene. The expression characteristics of an endogenous gene within a cell, e.g., a cell line or microorganism, can be modified by inserting a heterologous DNA regulatory element into the genome of the cell such that the inserted regulatory element is operably linked to the endogenous 69624 gene. For example, an endogenous 69624 gene which is "transcriptionally silent," e.g., not normally expressed, or expressed only at very low levels, may be activated by inserting a regulatory element which is capable of promoting the expression of a normally expressed gene product in that cell. Techniques such as targeted homologous recombinations, can be used to insert the heterologous DNA as described in, e.g., Chappel, US 5,272,071; WO 91/06667, published in May 16, 1991.

In a preferred embodiment, recombinant cells described herein can be used for replacement therapy in a subject. For example, a nucleic acid encoding a 69624 polypeptide operably linked to an inducible promoter (e.g., a steroid hormone receptor-regulated promoter) is introduced into a human or nonhuman, e.g., mammalian, e.g., porcine recombinant cell. The cell is cultivated and encapsulated in a biocompatible material, such as poly-lysine alginate, and subsequently implanted into the subject. See, e.g., Lanza (1996) *Nat. Biotechnol.* 14:1107; Joki *et al.* (2001) *Nat. Biotechnol.* 19:35; and U.S. Patent No. 5,876,742. Production of 69624 polypeptide can be regulated in the subject by administering an agent (e.g., a steroid hormone) to the subject. In another preferred embodiment, the implanted recombinant cells express and secrete an antibody specific for a 69624 polypeptide. The antibody can be any antibody or any antibody derivative described herein.

### Transgenic Animals

The invention provides non-human transgenic animals. Such animals are useful for studying the function and/or activity of a 69624 protein and for identifying and/or evaluating modulators of 69624 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, and the like. A transgene is exogenous DNA or a rearrangement, e.g., a deletion of endogenous chromosomal DNA, which preferably is integrated into or occurs in the genome of the cells of a transgenic animal. A transgene can direct the expression of an encoded gene product in one or more cell types or tissues of the transgenic animal, other transgenes, e.g., a knockout, reduce expression. Thus, a transgenic animal can be one in which an endogenous 69624 gene has been altered by, e.g., by homologous recombination between the endogenous gene and an exogenous DNA molecule introduced into a cell of the animal, e.g., an embryonic cell of the animal, prior to development of the animal.

Intronic sequences and polyadenylation signals can also be included in the transgene to increase the efficiency of expression of the transgene. A tissue-specific regulatory sequence(s) can be operably linked to a transgene of the invention to direct expression of a 69624 protein to particular cells. A transgenic founder animal can be identified based upon the presence of a 69624 transgene in its genome and/or expression of 69624 mRNA in tissues or cells of the animals. A transgenic founder animal can then be used to breed additional animals carrying the transgene. Moreover, transgenic animals carrying a transgene encoding a 69624 protein can further be bred to other transgenic animals carrying other transgenes.

69624 proteins or polypeptides can be expressed in transgenic animals or plants, e.g., a nucleic acid encoding the protein or polypeptide can be introduced into the genome of an animal. In preferred embodiments the nucleic acid is placed under the control of a tissue specific promoter, e.g., a milk or egg specific promoter, and recovered from the milk or eggs produced by the animal. Suitable animals are mice, pigs, cows, goats, and sheep.

The invention also includes a population of cells from a transgenic animal, as discussed, e.g., below.

### Uses

The nucleic acid molecules, proteins, protein homologues, and antibodies described herein can be used in one or more of the following methods: a) screening assays; b) predictive medicine (e.g., diagnostic assays, prognostic assays, monitoring clinical trials, and pharmacogenetics); and c) methods of treatment (e.g., therapeutic and prophylactic).

The isolated nucleic acid molecules of the invention can be used, for example, to express a 69624 protein (e.g., via a recombinant expression vector in a host cell in gene therapy applications), to detect a 69624 mRNA (e.g., in a biological sample) or a genetic alteration in a 69624 gene, and to modulate 69624 activity, as described further below. The 69624 proteins can be used to treat disorders characterized by insufficient or excessive production of a 69624 substrate or production of 69624 inhibitors. In addition, the 69624 proteins can be used to screen for naturally occurring 69624 substrates, to screen for drugs or compounds which modulate 69624 activity, as well as to treat disorders characterized by insufficient or excessive production of 69624 protein or production of 69624 protein forms which have decreased, aberrant or unwanted activity compared to 69624 wild type protein (e.g., disorders associated with renal or hepatic disfunction, or neural disorders). Moreover, the anti-69624 antibodies of the invention can be used to detect and isolate 69624 proteins, regulate the bioavailability of 69624 proteins, and modulate 69624 activity.

A method of evaluating a compound for the ability to interact with, e.g., bind, a subject 69624 polypeptide is provided. The method includes: contacting the compound with the subject 69624 polypeptide; and evaluating ability of the compound to interact with, e.g., to bind or form a complex with the subject 69624 polypeptide. This method can be performed in vitro, e.g., in a cell free system, or in vivo, e.g., in a two-hybrid interaction trap assay. This method can be used to identify naturally occurring molecules that interact with subject 69624 polypeptide. It can also be used to find natural or synthetic inhibitors of subject 69624 polypeptide. Screening methods are discussed in more detail below.

### Screening Assays

The invention provides methods (also referred to herein as "screening assays") for identifying modulators, i.e., candidate or test compounds or agents (e.g., proteins, peptides, peptidomimetics, peptoids, small molecules or other drugs) which bind to 69624 proteins, have a stimulatory or inhibitory effect on, for example, 69624 expression or 69624 activity, or have a stimulatory or inhibitory effect on, for example, the expression or activity of a 69624 substrate. Compounds thus identified can be used to modulate the activity of target gene products (e.g., 69624 genes) in a therapeutic protocol, to elaborate the biological function of the target gene product, or to identify compounds that disrupt normal target gene interactions.

In one embodiment, the invention provides assays for screening candidate or test compounds which are substrates of a 69624 protein or polypeptide or a biologically active portion thereof. In another embodiment, the invention provides assays for screening candidate or test compounds that bind to or modulate an activity of a 69624 protein or polypeptide or a biologically active portion thereof.

In one embodiment, an activity of a 69624 protein can be assayed by expressing the protein in an appropriate cell line and assaying for cellular uptake of a radiolabeled dicarboxylate ions, e.g., citrate or succinate, or radiolabeled sulfate ions present in the culture medium. Assays of this type have been described, for example, by Kekuda *et al.* (1999), *J Biol Chem* 274(6):3422-29, the contents of which are incorporated herein by reference.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann, R.N. *et al.* (1994) *J*. *Med. Chem.* 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) *Anticancer Drug Des.* 12:145).

Examples of methods for the synthesis of molecular libraries can be found in the art, for example in: DeWitt *et al.* (1993) *Proc. Natl. Acad. Sci. U.S.A.* 90:6909; Erb *et al.* (1994) *Proc. Natl. Acad. Sci. USA* 91:11422; Zuckermann *et al.* (1994). *J*. *Med. Chem.* 37:2678; Cho *et al.* (1993) *Science* 261:1303; Carrell *et al.* (1994) *Angew. Chem. Int. Ed. Engl.* 33:2059; Carell *et al.* (1994) *Angew. Chem. Int. Ed. Engl.* 33:2061; and Gallop *et al.* (1994) *J*. *Med. Chem.* 37:1233.

Libraries of compounds may be presented in solution (e.g., Houghten (1992) *Biotechniques* 13:412-421), or on beads (Lam (1991) *Nature* 354:82-84), chips (Fodor (1993) *Nature* 364:555-556), bacteria (Ladner, U.S. Patent No. 5,223,409), spores (Ladner U.S. Patent No. 5,223,409), plasmids (Cull *et al.* (1992) *Proc Natl Acad Sci USA* 89:1865-1869) or on phage (Scott and Smith (1990) *Science* 249:386-390; Devlin (1990) *Science* 249:404-406; Cwirla *et al.* (1990) *Proc. Natl. Acad. Sci.* 87:6378-6382; Felici (1991) *J*. *Mol. Biol.* 222:301-310; Ladner *supra*.).

In one embodiment, an assay is a cell-based assay in which a cell which expresses a 69624 protein or biologically active portion thereof is contacted with a test compound, and the ability of the test compound to modulate 69624 activity is determined. Determining the ability of the test compound to modulate 69624 activity can be accomplished by monitoring, for example, dicarboxylate or sulfate ion transport. The cell, for example, can be of mammalian origin, e.g., human.

The ability of the test compound to modulate 69624 binding to a compound, e.g., a 69624 substrate, or to bind to 69624 can also be evaluated. This can be accomplished, for example, by coupling the compound, e.g., the substrate, with a radioisotope or enzymatic label such that binding of the compound, e.g., the substrate, to 69624 can be determined by detecting the labeled compound, e.g., substrate, in a complex. Alternatively, 69624 could be coupled with a radioisotope or enzymatic label to monitor the ability of a test compound to modulate 69624 binding to a 69624 substrate in a complex. For example, compounds (e.g., 69624 substrates) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (e.g., a 69624 substrate) to interact with 69624 with or without the labeling of any of the interactants can be evaluated. For example, a microphysiometer can be used to detect the interaction of a compound with 69624 without the labeling of either the compound or the 69624. McConnell, H. M. *et al.* (1992) *Science* 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between a compound and 69624.

In yet another embodiment, a cell-free assay is provided in which a 69624 protein or biologically active portion thereof is contacted with a test compound and the ability of the test compound to bind to the 69624 protein or biologically active portion thereof is evaluated. Preferred biologically active portions of the 69624 proteins to be used in assays of the present invention include fragments which participate in interactions with non-69624 molecules, e.g., fragments with high surface probability scores.

Soluble and/or membrane-bound forms of isolated proteins (e.g., 69624 proteins or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy- 1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, e.g., using fluorescence energy transfer (FET) (see, for example, Lakowicz *et al*., U.S. Patent No. 5,631,169; Stavrianopoulos, *et al*., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determining the ability of the 69624 protein to bind to a target molecule can be accomplished using real-time Biomolecular Interaction Analysis (BIA) (see, e.g., Sjolander, S. and Urbaniczky, C. (1991) *Anal. Chem.* 63:2338-2345 and Szabo *et al.* (1995) *Curr*. *Opin*. *Struct*. *Biol*. 5:699-705). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 69624, an anti-69624 antibody or its target molecule to facilitate separation of complexed from uncomplexed forms of one or both of the proteins, as well as to accommodate automation of the assay. Binding of a test compound to a 69624 protein, or interaction of a 69624 protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase/69624 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 69624 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 69624 binding or activity determined using standard techniques.

Other techniques for immobilizing either a 69624 protein or a target molecule on matrices include using conjugation of biotin and streptavidin. Biotinylated 69624 protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with 69624 protein or target molecules but which do not interfere with binding of the 69624 protein to its target molecule. Such antibodies can be derivatized to the wells of the plate, and unbound target or 69624 protein trapped in the wells by antibody conjugation. Methods for detecting such complexes, in addition to those described above for the GST-immobilized complexes, include immunodetection of complexes using antibodies reactive with the 69624 protein or target molecule, as well as enzyme-linked assays which rely on detecting an enzymatic activity associated with the 69624 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas, G., and Minton, A.P., (1993) *Trends Biochem Sci* 18:284-7); chromatography (gel filtration chromatography, ion-exchange chromatography); electrophoresis (see, e.g., Ausubel, F. *et al*., eds. Current Protocols in Molecular Biology 1999, J. Wiley: New York.); and immunoprecipitation (see, for example, Ausubel, F. *et al.,* eds. (1999) *Current Protocols in Molecular Biology,* J. Wiley: New York). Such resins and chromatographic techniques are known to one skilled in the art (see, e.g., Heegaard, N.H., (1998) *J Mol Recognit* 11:141-8; Hage, D.S., and Tweed, S.A. (1997) *J Chromatogr B Biomed Sci Appl.* 699:499-525). Further, fluorescence energy transfer may also be conveniently utilized, as described herein, to detect binding without further purification of the complex from solution.

In a preferred embodiment, the assay includes contacting the 69624 protein or biologically active portion thereof with a known compound which binds 69624 to form an assay mixture, contacting the assay mixture with a test compound, and determining the ability of the test compound to interact with a 69624 protein, wherein determining the ability of the test compound to interact with a 69624 protein includes determining the ability of the test compound to preferentially bind to 69624 or biologically active portion thereof, or to modulate the activity of a target molecule, as compared to the known compound.

The target gene products of the invention can, *in vivo,* interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products for use in this embodiment are the 69624 genes herein identified. In an alternative embodiment, the invention provides methods for determining the ability of the test compound to modulate the activity of a 69624 protein through modulation of the activity of a downstream effector of a 69624 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 69624 proteins can be used as "bait proteins" in a two-hybrid assay or three-hybrid assay (see, e.g., U.S. Patent No. 5,283,317; Zervos *et al.* (1993) *Cell* 72:223-232; Madura *et al.* (1993) J *Biol*. *Chem.* 268:12046-12054; Bartel *et al.* (1993) *Biotechniques* 14:920-924; Iwabuchi *et al.* (1993) *Oncogene* 8:1693-1696; and Brent WO94/10300), to identify other proteins, which bind to or interact with 69624 ("69624-binding proteins" or "69624-bp") and are involved in 69624 activity. Such 69624-bps can be activators or inhibitors of signals by the 69624 proteins or 69624 targets as, for example, downstream elements of a 69624-mediated signaling pathway.

The two-hybrid system is based on the modular nature of most transcription factors, which consist of separable DNA-binding and activation domains. Briefly, the assay utilizes two different DNA constructs. In one construct, the gene that codes for a 69624 protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: 69624 protein can be the fused to the activator domain.) If the "bait" and the "prey" proteins are able to interact, *in vivo*, forming a 69624-dependent complex, the DNA-binding and activation domains of the transcription factor are brought into close proximity. This proximity allows transcription of a reporter gene (e.g., lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 69624 protein.

In another embodiment, modulators of 69624 expression are identified. For example, a cell or cell free mixture is contacted with a candidate compound and the expression of 69624 mRNA or protein evaluated relative to the level of expression of 69624 mRNA or protein in the absence of the candidate compound. When expression of 69624 mRNA or protein is greater in the presence of the candidate compound than in its absence, the candidate compound is identified as a stimulator of 69624 mRNA or protein expression. Alternatively, when expression of 69624 mRNA or protein is less (statistically significantly less) in the presence of the candidate compound than in its absence, the candidate compound is identified as an inhibitor of 69624 mRNA or protein expression. The level of 69624 mRNA or protein expression can be determined by methods described herein for detecting 69624 mRNA or protein.

In another aspect, the invention pertains to a combination of two or more of the assays described herein. For example, a modulating agent can be identified using a cell-based or a cell free assay, and the ability of the agent to modulate the activity of a 69624 protein can be confirmed *in vivo,* e.g., in an animal such as an animal model for renal of hepatic disfunction, or for a neural disorder.

This invention further pertains to novel agents identified by the above-described screening assays. Accordingly, it is within the scope of this invention to further use an agent identified as described herein (e.g., a 69624 modulating agent, an antisense 69624 nucleic acid molecule, a 69624-specific antibody, or a 69624-binding partner) in an appropriate animal model to determine the efficacy, toxicity, side effects, or mechanism of action, of treatment with such an agent. Furthermore, novel agents identified by the above-described screening assays can be used for treatments as described herein.

### Detection Assays

Portions or fragments of the nucleic acid sequences identified herein can be used as polynucleotide reagents. For example, these sequences can be used to: (i) map their respective genes on a chromosome e.g., to locate gene regions associated with genetic disease or to associate 69624 with a disease; (ii) identify an individual from a minute biological sample (tissue typing); and (iii) aid in forensic identification of a biological sample. These applications are described in the subsections below.

### Chromosome Mapping

The 69624 nucleotide sequences or portions thereof can be used to map the location of the 69624 genes on a chromosome. This process is called chromosome mapping. Chromosome mapping is useful in correlating the 69624 sequences with genes associated with disease.

Briefly, 69624 genes can be mapped to chromosomes by preparing PCR primers (preferably 15-25 bp in length) from the 69624 nucleotide sequences. These primers can then be used for PCR screening of somatic cell hybrids containing individual human chromosomes. Only those hybrids containing the human gene corresponding to the 69624 sequences will yield an amplified fragment.

A panel of somatic cell hybrids in which each cell line contains either a single human chromosome or a small number of human chromosomes, and a full set of mouse chromosomes, can allow easy mapping of individual genes to specific human chromosomes. (D'Eustachio P. *et al.* (1983) *Science* 220:919-924).

Other mapping strategies e.g., in situ hybridization (described in Fan, Y. *et al.* (1990) *Proc. Natl. Acad. Sci. USA*, 87:6223-27), pre-screening with labeled flow-sorted chromosomes, and pre-selection by hybridization to chromosome specific cDNA libraries can be used to map 69624 to a chromosomal location.

Fluorescence *in situ* hybridization (FISH) of a DNA sequence to a metaphase chromosomal spread can further be used to provide a precise chromosomal location in one step. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma *et al*., Human Chromosomes: A Manual of Basic Techniques ((1988) Pergamon Press, New York).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between a gene and a disease, mapped to the same chromosomal region, can then be identified through linkage analysis (co-inheritance of physically adjacent genes), described in, for example, Egeland, J. *et al.* (1987) *Nature,* 325:783-787. In one embodiment, the 69624 gene is mapped to human chromosome 17, so linkage analysis of 69624-associated diseases can map to regions of human chromosome 17.

Moreover, differences in the DNA sequences between individuals affected and unaffected with a disease associated with the 69624 gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

69624 sequences can be used to identify individuals from biological samples using, e.g., restriction fragment length polymorphism (RFLP). In this technique, an individual's genomic DNA is digested with one or more restriction enzymes, the fragments separated, e.g., in a Southern blot, and probed to yield bands for identification. The sequences of the present invention are useful as additional DNA markers for RFLP (described in U.S. Patent 5,272,057).

Furthermore, the sequences of the present invention can also be used to determine the actual base-by-base DNA sequence of selected portions of an individual's genome. Thus, the 69624 nucleotide sequences described herein can be used to prepare two PCR primers from the 5' and 3' ends of the sequences. These primers can then be used to amplify an individual's DNA and subsequently sequence it. Panels of corresponding DNA sequences from individuals, prepared in this manner, can provide unique individual identifications, as each individual will have a unique set of such DNA sequences due to allelic differences.

Allelic variation occurs to some degree in the coding regions of these sequences, and to a greater degree in the noncoding regions. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1 can provide positive individual identification with a panel of perhaps 10 to 1,000 primers which each yield a noncoding amplified sequence of 100 bases. If predicted coding sequences, such as those in SEQ ID NO:3 are used, a more appropriate number of primers for positive individual identification would be 500-2,000.

If a panel of reagents from 69624 nucleotide sequences described herein is used to generate a unique identification database for an individual, those same reagents can later be used to identify tissue from that individual. Using the unique identification database, positive identification of the individual, living or dead, can be made from extremely small tissue samples.

### Use of Partial 69624 Sequences in Forensic Biology

DNA-based identification techniques can also be used in forensic biology. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO:1 (e.g., fragments derived from the noncoding regions of SEQ ID NO:1 having a length of at least 20 bases, preferably at least 30 bases) are particularly appropriate for this use.

The 69624 nucleotide sequences described herein can further be used to provide polynucleotide reagents, e.g., labeled or labelable probes which can be used in, for example, an *in situ* hybridization technique, to identify a specific tissue. This can be very useful in cases where a forensic pathologist is presented with a tissue of unknown origin. Panels of such 69624 probes can be used to identify tissue by species and/or by organ type.

In a similar fashion, these reagents, e.g., 69624 primers or probes can be used to screen tissue culture for contamination (i.e. screen for the presence of a mixture of different types of cells in a culture).

### Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 69624.

Such disorders include, e.g., a disorder associated with the misexpression of 69624 gene, or a disorder of the kidney, liver, or brain.

The method includes one or more of the following:

detecting, in a tissue of the subject, the presence or absence of a mutation which affects the expression of the 69624 gene, or detecting the presence or absence of a mutation in a region which controls the expression of the gene, e.g., a mutation in the 5' control region;

detecting, in a tissue of the subject, the presence or absence of a mutation which alters the structure of the 69624 gene;

detecting, in a tissue of the subject, the misexpression of the 69624 gene, at the mRNA level, e.g., detecting a non-wild type level of a mRNA ;

detecting, in a tissue of the subject, the misexpression of the gene, at the protein level, e.g., detecting a non-wild type level of a 69624 polypeptide.

In preferred embodiments the method includes: ascertaining the existence of at least one of: a deletion of one or more nucleotides from the 69624 gene; an insertion of one or more nucleotides into the gene, a point mutation, e.g., a substitution of one or more nucleotides of the gene, a gross chromosomal rearrangement of the gene, e.g., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1, or naturally occurring mutants thereof or 5' or 3' flanking sequences naturally associated with the 69624 gene; (ii) exposing the probe/primer to nucleic acid of the tissue; and detecting, by hybridization, e.g., *in situ* hybridization, of the probe/primer to the nucleic acid, the presence or absence of the genetic lesion.

In preferred embodiments, detecting the misexpression includes ascertaining the existence of at least one of: an alteration in the level of a messenger RNA transcript of the 69624 gene; the presence of a non-wild type splicing pattern of a messenger RNA transcript of the gene; or a non-wild type level of 69624.

Methods of the invention can be used prenatally or to determine if a subject's offspring will be at risk for a disorder.

In preferred embodiments the method includes determining the structure of a 69624 gene, an abnormal structure being indicative of risk for the disorder.

In preferred embodiments the method includes contacting a sample from the subject with an antibody to the 69624 protein or a nucleic acid, which hybridizes specifically with the gene. These and other embodiments are discussed below.

### Diagnostic and Prognostic Assays

Diagnostic and prognostic assays of the invention include method for assessing the expression level of 69624 molecules and for identifying variations and mutations in the sequence of 69624 molecules.

### Expression Monitoring and Profiling

The presence, level, or absence of 69624 protein or nucleic acid in a biological sample can be evaluated by obtaining a biological sample from a test subject and contacting the biological sample with a compound or an agent capable of detecting 69624 protein or nucleic acid (e.g., mRNA, genomic DNA) that encodes 69624 protein such that the presence of 69624 protein or nucleic acid is detected in the biological sample. The term "biological sample" includes tissues, cells and biological fluids isolated from a subject, as well as tissues, cells and fluids present within a subject. A preferred biological sample is serum. The level of expression of the 69624 gene can be measured in a number of ways, including, but not limited to: measuring the mRNA encoded by the 69624 genes; measuring the amount of protein encoded by the 69624 genes; or measuring the activity of the protein encoded by the 69624 genes.

The level of mRNA corresponding to the 69624 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 69624 nucleic acid, such as the nucleic acid of SEQ ID NO:1, or a portion thereof, such as an oligonucleotide of at least 7, 15, 30, 50, 100, 250 or 500 nucleotides in length and sufficient to specifically hybridize under stringent conditions to 69624 mRNA or genomic DNA. The probe can be disposed on an address of an array, e.g., an array described below. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array described below. A skilled artisan can adapt known mRNA detection methods for use in detecting the level of mRNA encoded by the 69624 genes.

The level of mRNA in a sample that is encoded by one of 69624 can be evaluated with nucleic acid amplification, e.g., by rtPCR (Mullis (1987) U.S. Patent No. 4,683,202), ligase chain reaction (Barany (1991) *Proc. Natl. Acad. Sci. USA* 88:189-193), self sustained sequence replication (Guatelli *et al*., (1990) *Proc. Natl. Acad. Sci. USA* 87:1874-1878), transcriptional amplification system (Kwoh *et al.,* (1989), *Proc. Natl. Acad. Sci. USA* 86:1173-1177), Q-Beta Replicase (Lizardi *et al*., (1988) *Bio*/*Technology* 6:1197), rolling circle replication (Lizardi *et al*., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 69624 gene being analyzed.

In another embodiment, the methods further contacting a control sample with a compound or agent capable of detecting 69624 mRNA, or genomic DNA, and comparing the presence of 69624 mRNA or genomic DNA in the control sample with the presence of 69624 mRNA or genomic DNA in the test sample. In still another embodiment, serial analysis of gene expression, as described in U.S. Patent No. 5,695,937, is used to detect 69624 transcript levels.

A variety of methods can be used to determine the level of protein encoded by 69624. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Examples of detectable substances are provided herein.

The detection methods can be used to detect 69624 protein in a biological sample *in vitro* as well as *in vivo. In vitro* techniques for detection of 69624 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 69624 protein include introducing into a subject a labeled anti-69624 antibody. For example, the antibody can be labeled with a radioactive marker whose presence and location in a subject can be detected by standard imaging techniques. In another embodiment, the sample is labeled, e.g., biotinylated and then contacted to the antibody, e.g., an anti-69624 antibody positioned on an antibody array (as described below). The sample can be detected, e.g., with avidin coupled to a fluorescent label.

In another embodiment, the methods further include contacting the control sample with a compound or agent capable of detecting 69624 protein, and comparing the presence of 69624 protein in the control sample with the presence of 69624 protein in the test sample.

The invention also includes kits for detecting the presence of 69624 in a biological sample. For example, the kit can include a compound or agent capable of detecting 69624 protein or mRNA in a biological sample; and a standard. The compound or agent can be packaged in a suitable container. The kit can further comprise instructions for using the kit to detect 69624 protein or nucleic acid.

For antibody-based kits, the kit can include: (1) a first antibody (e.g., attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, e.g., a detectably labeled oligonucleotide, which hybridizes to a nucleic acid sequence encoding a polypeptide corresponding to a marker of the invention or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (e.g., an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 69624 expression or activity. As used herein, the term "unwanted" includes an unwanted phenomenon involved in a biological response such as renal, hepatic, or intestinal disfunction, or a neural disorder.

In one embodiment, a disease or disorder associated with aberrant or unwanted 69624 expression or activity is identified. A test sample is obtained from a subject and 69624 protein or nucleic acid (e.g., mRNA or genomic DNA) is evaluated, wherein the level, e.g., the presence or absence, of 69624 protein or nucleic acid is diagnostic for a subject having or at risk of developing a disease or disorder associated with aberrant or unwanted 69624 expression or activity. As used herein, a "test sample" refers to a biological sample obtained from a subject of interest, including a biological fluid (e.g., serum), cell sample, or tissue.

The prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 69624 expression or activity. For example, such methods can be used to determine whether a subject can be effectively treated with an agent for a renal, hepatic, intestinal, or neural disorder.

In another aspect, the invention features a computer medium having a plurality of digitally encoded data records. Each data record includes a value representing the level of expression of 69624 in a sample, and a descriptor of the sample. The descriptor of the sample can be an identifier of the sample, a subject from which the sample was derived (e.g., a patient), a diagnosis, or a treatment (e.g., a preferred treatment). In a preferred embodiment, the data record further includes values representing the level of expression of genes other than 69624 (e.g., other genes associated with a 69624-disorder, or other genes on an array). The data record can be structured as a table, e.g., a table that is part of a database such as a relational database (e.g., a SQL database of the Oracle or Sybase database environments).

Also featured is a method of evaluating a sample. The method includes providing a sample, e.g., from the subject, and determining a gene expression profile of the sample, wherein the profile includes a value representing the level of 69624 expression. The method can further include comparing the value or the profile (i.e., multiple values) to a reference value or reference profile. The gene expression profile of the sample can be obtained by any of the methods described herein (e.g., by providing a nucleic acid from the sample and contacting the nucleic acid to an array). The method can be used to diagnose a renal, hepatic, or neural disorder in a subject wherein a decrease in 69624 expression is an indication that the subject has or is disposed to having such a disorder. The method can be used to monitor a treatment for a renal, hepatic, or neural disorder in a subject. For example, the gene expression profile can be determined for a sample from a subject undergoing treatment. The profile can be compared to a reference profile or to a profile obtained from the subject prior to treatment or prior to onset of the disorder (see, e.g., Golub *et al.* (1999) *Science* 286:531).

In yet another aspect, the invention features a method of evaluating a test compound (see also, "Screening Assays", above). The method includes providing a cell and a test compound; contacting the test compound to the cell; obtaining a subject expression profile for the contacted cell; and comparing the subject expression profile to one or more reference profiles. The profiles include a value representing the level of 69624 expression. In a preferred embodiment, the subject expression profile is compared to a target profile, e.g., a profile for a normal cell or for desired condition of a cell. The test compound is evaluated favorably if the subject expression profile is more similar to the target profile than an expression profile obtained from an uncontacted cell.

In another aspect, the invention features, a method of evaluating a subject. The method includes: a) obtaining a sample from a subject, e.g., from a caregiver, e.g., a caregiver who obtains the sample from the subject; b) determining a subject expression profile for the sample. Optionally, the method further includes either or both of steps: c) comparing the subject expression profile to one or more reference expression profiles; and d) selecting the reference profile most similar to the subject reference profile. The subject expression profile and the reference profiles include a value representing the level of 69624 expression. A variety of routine statistical measures can be used to compare two reference profiles. One possible metric is the length of the distance vector that is the difference between the two profiles. Each of the subject and reference profile is represented as a multidimensional vector, wherein each dimension is a value in the profile.

The method can further include transmitting a result to a caregiver. The result can be the subject expression profile, a result of a comparison of the subject expression profile with another profile, a most similar reference profile, or a descriptor of any of the aforementioned. The result can be transmitted across a computer network, e.g., the result can be in the form of a computer transmission, e.g., a computer data signal embedded in a carrier wave.

Also featured is a computer medium having executable code for effecting the following steps: receive a subject expression profile; access a database of reference expression profiles; and either i) select a matching reference profile most similar to the subject expression profile or ii) determine at least one comparison score for the similarity of the subject expression profile to at least one reference profile. The subject expression profile, and the reference expression profiles each include a value representing the level of 69624 expression.

### Arrays and Uses Thereof

In another aspect, the invention features an array that includes a substrate having a plurality of addresses. At least one address of the plurality includes a capture probe that binds specifically to a 69624 molecule (e.g., a 69624 nucleic acid or a 69624 polypeptide). The array can have a density of at least than 10, 50, 100, 200, 500, 1,000, 2,000, or 10,000 or more addresses/cm², and ranges between. In a preferred embodiment, the plurality of addresses includes at least 10, 100, 500, 1,000, 5,000, 10,000, 50,000 addresses. In a preferred embodiment, the plurality of addresses includes equal to or less than 10, 100, 500, 1,000, 5,000, 10,000, or 50,000 addresses. The substrate can be a two-dimensional substrate such as a glass slide, a wafer (e.g., silica or plastic), a mass spectroscopy plate, or a three-dimensional substrate such as a gel pad. Addresses in addition to address of the plurality can be disposed on the array.

In a preferred embodiment, at least one address of the plurality includes a nucleic acid capture probe that hybridizes specifically to a 69624 nucleic acid, e.g., the sense or anti-sense strand. In one preferred embodiment, a subset of addresses of the plurality of addresses has a nucleic acid capture probe for 69624. Each address of the subset can include a capture probe that hybridizes to a different region of a 69624 nucleic acid. In another preferred embodiment, addresses of the subset include a capture probe for a 69624 nucleic acid. Each address of the subset is unique, overlapping, and complementary to a different variant of 69624 (e.g., an allelic variant, or all possible hypothetical variants). The array can be used to sequence 69624 by hybridization (see, e.g., U.S. Patent No. 5,695,940).

An array can be generated by various methods, e.g., by photolithographic methods (see, e.g., U.S. Patent Nos. 5,143,854; 5,510,270; and 5,527,681), mechanical methods (e.g., directed-flow methods as described in U.S. Patent No. 5,384,261), pin-based methods (e.g., as described in U.S. Pat. No. 5,288,514), and bead-based techniques (e.g., as described in PCT US/93/04145).

In another preferred embodiment, at least one address of the plurality includes a polypeptide capture probe that binds specifically to a 69624 polypeptide or fragment thereof. The polypeptide can be a naturally-occurring interaction partner of 69624 polypeptide. Preferably, the polypeptide is an antibody, e.g., an antibody described herein (see "Anti-69624 Antibodies," above), such as a monoclonal antibody or a single-chain antibody.

In another aspect, the invention features a method of analyzing the expression of 69624. The method includes providing an array as described above; contacting the array with a sample and detecting binding of a 69624-molecule (e.g., nucleic acid or polypeptide) to the array. In a preferred embodiment, the array is a nucleic acid array. Optionally, the method further includes amplifying nucleic acid from the sample prior or during contact with the array.

In another embodiment, the array can be used to assay gene expression in a tissue to ascertain tissue specificity of genes in the array, particularly the expression of 69624. If a sufficient number of diverse samples is analyzed, clustering (e.g., hierarchical clustering, k-means clustering, Bayesian clustering and the like) can be used to identify other genes which are co-regulated with 69624. For example, the array can be used for the quantitation of the expression of multiple genes. Thus, not only tissue specificity, but also the level of expression of a battery of genes in the tissue is ascertained. Quantitative data can be used to group (e.g., cluster) genes on the basis of their tissue expression per *se* and level of expression in that tissue.

For example, array analysis of gene expression can be used to assess the effect of cell-cell interactions on 69624 expression. A first tissue can be perturbed and nucleic acid from a second tissue that interacts with the first tissue can be analyzed. In this context, the effect of one cell type on another cell type in response to a biological stimulus can be determined, e.g., to monitor the effect of cell-cell interaction at the level of gene expression.

In another embodiment, cells are contacted with a therapeutic agent. The expression profile of the cells is determined using the array, and the expression profile is compared to the profile of like cells not contacted with the agent. For example, the assay can be used to determine or analyze the molecular basis of an undesirable effect of the therapeutic agent. If an agent is administered therapeutically to treat one cell type but has an undesirable effect on another cell type, the invention provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor expression of one or more genes in the array with respect to time. For example, samples obtained from different time points can be probed with the array. Such analysis can identify and/or characterize the development of a 69624-associated disease or disorder; and processes, such as a cellular transformation associated with a 69624-associated disease or disorder. The method can also evaluate the treatment and/or progression of a 69624-associated disease or disorder

The array is also useful for ascertaining differential expression patterns of one or more genes in normal and abnormal cells. This provides a battery of genes (*e.g*., including 69624) that could serve as a molecular target for diagnosis or therapeutic intervention.

In another aspect, the invention features an array having a plurality of addresses. Each address of the plurality includes a unique polypeptide. At least one address of the plurality has disposed thereon a 69624 polypeptide or fragment thereof. Methods of producing polypeptide arrays are described in the art, e.g., in De Wildt *et al.* (2000). *Nature Biotech. 18,* 989-994; Lueking *et al.* (1999). *Anal. Biochem. 270*, 103-111; Ge, H. (2000). *Nucleic Acids Res. 28,* e3, I-VII; MacBeath, G., and Schreiber, S.L. (2000). *Science 289,* 1760-1763; and WO 99/51773A1. In a preferred embodiment, each addresses of the plurality has disposed thereon a polypeptide at least 60, 70, 80,85, 90, 95 or 99 % identical to a 69624 polypeptide or fragment thereof. For example, multiple variants of a 69624 polypeptide (e.g., encoded by allelic variants, site-directed mutants, random mutants, or combinatorial mutants) can be disposed at individual addresses of the plurality. Addresses in addition to the address of the plurality can be disposed on the array.

The polypeptide array can be used to detect a 69624 binding compound, e.g., an antibody in a sample from a subject with specificity for a 69624 polypeptide or the presence of a 69624-binding protein or ligand.

The array is also useful for ascertaining the effect of the expression of a gene on the expression of other genes in the same cell or in different cells (*e.g*., ascertaining the effect of 69624 expression on the expression of other genes). This provides, for example, for a selection of alternate molecular targets for therapeutic intervention if the ultimate or downstream target cannot be regulated.

In another aspect, the invention features a method of analyzing a plurality of probes. The method is useful, e.g., for analyzing gene expression. The method includes: providing a two dimensional array having a plurality of addresses, each address of the plurality being positionally distinguishable from each other address of the plurality having a unique capture probe, e.g., wherein the capture probes are from a cell or subject which express 69624 or from a cell or subject in which a 69624 mediated response has been elicited, e.g., by contact of the cell with 69624 nucleic acid or protein, or administration to the cell or subject 69624 nucleic acid or protein; providing a two dimensional array having a plurality of addresses, each address of the plurality being positionally distinguishable from each other address of the plurality, and each address of the plurality having a unique capture probe, e.g., wherein the capture probes are from a cell or subject which does not express 69624 (or does not express as highly as in the case of the 69624 positive plurality of capture probes) or from a cell or subject which in which a 69624 mediated response has not been elicited (or has been elicited to a lesser extent than in the first sample); contacting the array with one or more inquiry probes (which is preferably other than a 69624 nucleic acid, polypeptide, or antibody), and thereby evaluating the plurality of capture probes. Binding, e.g., in the case of a nucleic acid, hybridization with a capture probe at an address of the plurality, is detected, e.g., by signal generated from a label attached to the nucleic acid, polypeptide, or antibody.

In another aspect, the invention features a method of analyzing a plurality of probes or a sample. The method is useful, e.g., for analyzing gene expression. The method includes: providing a two dimensional array having a plurality of addresses, each address of the plurality being positionally distinguishable from each other address of the plurality having a unique capture probe, contacting the array with a first sample from a cell or subject which express or mis-express 69624 or from a cell or subject in which a 69624-mediated response has been elicited, e.g., by contact of the cell with 69624 nucleic acid or protein, or administration to the cell or subject 69624 nucleic acid or protein; providing a two dimensional array having a plurality of addresses, each address of the plurality being positionally distinguishable from each other address of the plurality, and each address of the plurality having a unique capture probe, and contacting the array with a second sample from a cell or subject which does not express 69624 (or does not express as highly as in the case of the 69624 positive plurality of capture probes) or from a cell or subject which in which a 69624 mediated response has not been elicited (or has been elicited to a lesser extent than in the first sample); and comparing the binding of the first sample with the binding of the second sample. Binding, e.g., in the case of a nucleic acid, hybridization with a capture probe at an address of the plurality, is detected, e.g., by signal generated from a label attached to the nucleic acid, polypeptide, or antibody. The same array can be used for both samples or different arrays can be used. If different arrays are used the plurality of addresses with capture probes should be present on both arrays.

In another aspect, the invention features a method of analyzing 69624, e.g., analyzing structure, function, or relatedness to other nucleic acid or amino acid sequences. The method includes: providing a 69624 nucleic acid or amino acid sequence; comparing the 69624 sequence with one or more preferably a plurality of sequences from a collection of sequences, e.g., a nucleic acid or protein sequence database; to thereby analyze 69624.

### Detection of Variations or Mutations

The methods of the invention can also be used to detect genetic alterations in a 69624 gene, thereby determining if a subject with the altered gene is at risk for a disorder characterized by misregulation in 69624 protein activity or nucleic acid expression, such as a renal, hepatic, or neural disorder. In preferred embodiments, the methods include detecting, in a sample from the subject, the presence or absence of a genetic alteration characterized by at least one of an alteration affecting the integrity of a gene encoding a 69624-protein, or the mis-expression of the 69624 gene. For example, such genetic alterations can be detected by ascertaining the existence of at least one of 1) a deletion of one or more nucleotides from a 69624 gene; 2) an addition of one or more nucleotides to a 69624 gene; 3) a substitution of one or more nucleotides of a 69624 gene, 4) a chromosomal rearrangement of a 69624 gene; 5) an alteration in the level of a messenger RNA transcript of a 69624 gene, 6) aberrant modification of a 69624 gene, such as of the methylation pattern of the genomic DNA, 7) the presence of a non-wild type splicing pattern of a messenger RNA transcript of a 69624 gene, 8) a non-wild type level of a 69624-protein, 9) allelic loss of a 69624 gene, and 10) inappropriate post-translational modification of a 69624-protein.

An alteration can be detected without a probe/primer in a polymerase chain reaction, such as anchor PCR or RACE PCR, or, alternatively, in a ligation chain reaction (LCR), the latter of which can be particularly useful for detecting point mutations in the 69624-gene. This method can include the steps of collecting a sample of cells from a subject, isolating nucleic acid (e.g., genomic, mRNA or both) from the sample, contacting the nucleic acid sample with one or more primers which specifically hybridize to a 69624 gene under conditions such that hybridization and amplification of the 69624-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein. Alternatively, other amplification methods described herein or known in the art can be used.

In another embodiment, mutations in a 69624 gene from a sample cell can be identified by detecting alterations in restriction enzyme cleavage patterns. For example, sample and control DNA is isolated, amplified (optionally), digested with one or more restriction endonucleases, and fragment length sizes are determined, e.g., by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 69624 can be identified by hybridizing a sample and control nucleic acids, e.g., DNA or RNA, two-dimensional arrays, e.g., chip based arrays. Such arrays include a plurality of addresses, each of which is positionally distinguishable from the other. A different probe is located at each address of the plurality. A probe can be complementary to a region of a 69624 nucleic acid or a putative variant (e.g., allelic variant) thereof. A probe can have one or more mismatches to a region of a 69624 nucleic acid (e.g., a destabilizing mismatch). The arrays can have a high density of addresses, e.g., can contain hundreds or thousands of oligonucleotides probes (Cronin, M.T. *et al.* (1996) *Human Mutation* 7: 244-255; Kozal, M.J. *et al.* (1996) *Nature Medicine* 2: 753-759). For example, genetic mutations in 69624 can be identified in two-dimensional arrays containing light-generated DNA probes as described in Cronin, M.T. *et al. supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 69624 gene and detect mutations by comparing the sequence of the sample 69624 with the corresponding wild-type (control) sequence. Automated sequencing procedures can be utilized when performing the diagnostic assays ((1995) *Biotechniques* 19:448), including sequencing by mass spectrometry.

Other methods for detecting mutations in the 69624 gene include methods in which protection from cleavage agents is used to detect mismatched bases in RNA/RNA or RNA/DNA heteroduplexes (Myers *et al.* (1985) *Science* 230:1242; Cotton *et al.* (1988) *Proc. Natl Acad Sci USA* 85:4397; Saleeba *et al.* (1992) *Methods Enzymol.* 217:286-295).

In still another embodiment, the mismatch cleavage reaction employs one or more proteins that recognize mismatched base pairs in double-stranded DNA (so called "DNA mismatch repair" enzymes) in defined systems for detecting and mapping point mutations in 69624 cDNAs obtained from samples of cells. For example, the mutY enzyme of *E*. *coli* cleaves A at G/A mismatches and the thymidine DNA glycosylase from HeLa cells cleaves T at G/T mismatches (Hsu *et al.* (1994) *Carcinogenesis* 15:1657-1662; U.S. Patent No. 5,459,039).

In other embodiments, alterations in electrophoretic mobility will be used to identify mutations in 69624 genes. For example, single strand conformation polymorphism (SSCP) may be used to detect differences in electrophoretic mobility between mutant and wild type nucleic acids (Orita *et al.* (1989) *Proc Natl. Acad. Sci USA*: 86:2766, see also Cotton (1993) *Mutat. Res.* 285:125-144; and Hayashi (1992) *Genet. Anal. Tech. Appl.* 9:73-79). Single-stranded DNA fragments of sample and control 69624 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen *et al.* (1991) *Trends Genet* 7:5).

In yet another embodiment, the movement of mutant or wild-type fragments in polyacrylamide gels containing a gradient of denaturant is assayed using denaturing gradient gel electrophoresis (DGGE) (Myers *et al.* (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys Chem* 265:12753).

Examples of other techniques for detecting point mutations include, but are not limited to, selective oligonucleotide hybridization, selective amplification, or selective primer extension (Saiki *et al.* (1986) *Nature* 324:163); Saiki *et al.* (1989) *Proc. Natl Acad Sci USA* 86:6230). A further method of detecting point mutations is the chemical ligation of oligonucleotides as described in Xu *et al.* ((2001) *Nature Biotechnol.* 19:148). Adjacent oligonucleotides, one of which selectively anneals to the query site, are ligated together if the nucleotide at the query site of the sample nucleic acid is complementary to the query oligonucleotide; ligation can be monitored, e.g., by fluorescent dyes coupled to the oligonucleotides.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization) (Gibbs *et al.* (1989) *Nucleic Acids Res.* 17:2437-2448) or at the extreme 3' end of one primer where, under appropriate conditions, mismatch can prevent, or reduce polymerase extension (Prossner (1993) *Tibtech* 11:238). In addition it may be desirable to introduce a novel restriction site in the region of the mutation to create cleavage-based detection (Gasparini *et al.* (1992) *Mol. Cell Probes* 6:1). It is anticipated that in certain embodiments amplification may also be performed using Taq ligase for amplification (Barany (1991) *Proc. Natl. Acad. Sci USA* 88:189). In such cases, ligation will occur only if there is a perfect match at the 3' end of the 5' sequence making it possible to detect the presence of a known mutation at a specific site by looking for the presence or absence of amplification.

In another aspect, the invention features a set of oligonucleotides. The set includes a plurality of oligonucleotides, each of which is at least partially complementary (e.g., at least 50%, 60%, 70%, 80%, 90%, 92%, 95%, 97%, 98%, or 99% complementary) to a 69624 nucleic acid.

In a preferred embodiment the set includes a first and a second oligonucleotide. The first and second oligonucleotide can hybridize to the same or to different locations of SEQ ID NO: 1 or the complement of SEQ ID NO: 1. Different locations can be different but overlapping, or non-overlapping on the same strand. The first and second oligonucleotide can hybridize to sites on the same or on different strands.

The set can be useful, e.g., for identifying SNP's, or identifying specific alleles of 69624. In a preferred embodiment, each oligonucleotide of the set has a different nucleotide at an interrogation position. In one embodiment, the set includes two oligonucleotides, each complementary to a different allele at a locus, e.g., a biallelic or polymorphic locus.

In another embodiment, the set includes four oligonucleotides, each having a different nucleotide (e.g., adenine, guanine, cytosine, or thymidine) at the interrogation position. The interrogation position can be a SNP or the site of a mutation. In another preferred embodiment, the oligonucleotides of the plurality are identical in sequence to one another (except for differences in length). The oligonucleotides can be provided with differential labels, such that an oligonucleotide that hybridizes to one allele provides a signal that is distinguishable from an oligonucleotide that hybridizes to a second allele. In still another embodiment, at least one of the oligonucleotides of the set has a nucleotide change at a position in addition to a query position, e.g., a destabilizing mutation to decrease the Tₘ of the oligonucleotide. In another embodiment, at least one oligonucleotide of the set has a non-natural nucleotide, e.g., inosine. In a preferred embodiment, the oligonucleotides are attached to a solid support, e.g., to different addresses of an array or to different beads or nanoparticles.

In a preferred embodiment the set of oligo nucleotides can be used to specifically amplify, e.g., by PCR, or detect, a 69624 nucleic acid.

The methods described herein may be performed, for example, by utilizing pre-packaged diagnostic kits comprising at least one probe nucleic acid or antibody reagent described herein, which may be conveniently used, *e.g*., in clinical settings to diagnose patients exhibiting symptoms or family history of a disease or illness involving a 69624 gene.

### Use of 69624 Molecules as Surrogate Markers

The 69624 molecules of the invention are also useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity of the 69624 molecules of the invention may be detected, and may be correlated with one or more biological states in vivo. For example, the 69624 molecules of the invention may serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (e.g., with the presence or absence of a tumor). The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies (e.g., early stage tumors), or when an assessment of disease progression is desired before a potentially dangerous clinical endpoint is reached (e.g., an assessment of cardiovascular disease may be made using cholesterol levels as a surrogate marker, and an analysis of HIV infection may be made using HIV RNA levels as a surrogate marker, well in advance of the undesirable clinical outcomes of myocardial infarction or fully-developed AIDS). Examples of the use of surrogate markers in the art include: Koomen *et al.* (2000) *J. Mass. Spectrom.* 35: 258-264; and James (1994) *AIDS Treatment News Archive* 209.

The 69624 molecules of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug in vivo. Pharmacodynamic markers are of particular use in increasing the sensitivity of detection of drug effects, particularly when the drug is administered in low doses. Since even a small amount of a drug may be sufficient to activate multiple rounds of marker (e.g., a 69624 marker) transcription or expression, the amplified marker may be in a quantity which is more readily detectable than the drug itself. Also, the marker may be more easily detected due to the nature of the marker itself; for example, using the methods described herein, anti-69624 antibodies may be employed in an immune-based detection system for a 69624 protein marker, or 69624-specific radiolabeled probes may be used to detect a 69624 mRNA marker. Furthermore, the use of a pharmacodynamic marker may offer mechanism-based prediction of risk due to drug treatment beyond the range of possible direct observations. Examples of the use of pharmacodynamic markers in the art include: Matsuda *et al.* US 6,033,862; Hattis *et al.* (1991) *Env. Health Perspect.* 90: 229-238; Schentag (1999) *Am. J Health-Syst. Pharm.* 56 Suppl. 3: S21-S24; and Nicolau (1999) *Am, J. Health-Syst. Pharm.* 56 Suppl. 3: S16-S20.

The 69624 molecules of the invention are also useful as pharmacogenomic markers. As used herein, a "pharmacogenomic marker" is an objective biochemical marker which correlates with a specific clinical drug response or susceptibility in a subject (see, e.g., McLeod *et al.* (1999) *Eur. J*. *Cancer* 35:1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, may be selected. For example, based on the presence or quantity of RNA, or protein (e.g., 69624 protein or RNA) for specific tumor markers in a subject, a drug or course of treatment may be selected that is optimized for the treatment of the specific tumor likely to be present in the subject. Similarly, the presence or absence of a specific sequence mutation in 69624 DNA may correlate 69624 drug response. The use of pharmacogenomic markers therefore permits the application of the most appropriate treatment for each subject without having to administer the therapy.

### Pharmaceutical Compositions

The nucleic acid and polypeptides, fragments thereof, as well as anti-69624 antibodies (also referred to herein as "active compounds") of the invention can be incorporated into pharmaceutical compositions. Such compositions typically include the nucleic acid molecule, protein, or antibody and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

For antibodies, the preferred dosage is 0.1 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (e.g., into the brain). A method for lipidation of antibodies is described by Cruikshank *et al.* ((1997) *J*. *Acquired Immune Deficiency Syndromes and Human Retrovirology* 14:193).

The present invention encompasses agents which modulate expression or activity. An agent may, for example, be a small molecule. For example, such small molecules include, but are not limited to, peptides, peptidomimetics (e.g., peptoids), amino acids, amino acid analogs, polynucleotides, polynucleotide analogs, nucleotides, nucleotide analogs, organic or inorganic compounds (i.e.,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

An antibody (or fragment thereof) may be conjugated to a therapeutic moiety such as a cytotoxin, a therapeutic agent or a radioactive ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids, e.g., maytansinol (see US Patent No. 5,208,020), CC-1065 (see US Patent Nos. 5,475,092, 5,585,499, 5,846,545) and analogs or homologs thereof. Therapeutic agents include, but are not limited to, antimetabolites (*e.g*., methotrexate, 6-mercaptopurine, 6-thioguanine, cytarabine, 5-fluorouracil decarbazine), alkylating agents (*e.g*., mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g*., daunorubicin (formerly daunomycin) and doxorubicin), antibiotics (*e.g*., dactinomycin (formerly actinomycin), bleomycin, mithramycin, and anthramycin (AMC)), and anti-mitotic agents *(e.g.,* vincristine, vinblastine, taxol and maytansinoids). Radioactive ions include, but are not limited to iodine, yttrium and praseodymium.

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Alternatively, an antibody can be conjugated to a second antibody to form an antibody heteroconjugate as described by Segal in U.S. Patent No. 4,676,980.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen *et al.* (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### Methods of Treatment

The present invention provides for both prophylactic and therapeutic methods of treating a subject at risk of (or susceptible to) a disorder or having a disorder associated with aberrant or unwanted 69624 expression or activity. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes and antisense oligonucleotides.

With regards to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype".) Thus, another aspect of the invention provides methods for tailoring an individual's prophylactic or therapeutic treatment with either the 69624 molecules of the present invention or 69624 modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

In one aspect, the invention provides a method for preventing in a subject, a disease or condition associated with an aberrant or unwanted 69624 expression or activity, by administering to the subject a 69624 or an agent which modulates 69624 expression or at least one 69624 activity. Subjects at risk for a disease which is caused or contributed to by aberrant or unwanted 69624 expression or activity can be identified by, for example, any or a combination of diagnostic or prognostic assays as described herein. Administration of a prophylactic agent can occur prior to the manifestation of symptoms characteristic of the 69624 aberrance, such that a disease or disorder is prevented or, alternatively, delayed in its progression. Depending on the type of 69624 aberrance, for example, a 69624, 69624 agonist or 69624 antagonist agent can be used for treating the subject. The appropriate agent can be determined based on screening assays described herein.

It is possible that some 69624 disorders can be caused, at least in part, by an abnormal level of gene product, or by the presence of a gene product exhibiting abnormal activity. As such, the reduction in the level and/or activity of such gene products would bring about the amelioration of disorder symptoms.

The 69624 molecules can act as novel diagnostic targets and therapeutic agents for controlling disorders of the kidney, brain, colon, liver, salivary gland, as well as metabolic disorders, as discussed above. In addition, the 69624 molecules may act as novel diagnostic targets and therapeutic agents for controlling cellular proliferative and/or differentiative disorders, immune disorders, cardiovascular disorders, and disorders involving abnormal or excessive pain.

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin.

As used herein, the terms "cancer", "hyperproliferative" and "neoplastic" refer to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

The terms "cancer" or "neoplasms" include malignancies of the various organ systems, such as affecting lung, breast, thyroid, lymphoid, gastrointestinal, and genitourinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

Additional examples of proliferative disorders include hematopoietic neoplastic disorders. As used herein, the term "hematopoietic neoplastic disorders" includes diseases involving hyperplastic/neoplastic cells of hematopoietic origin, e.g., arising from myeloid, lymphoid or erythroid lineages, or precursor cells thereof. Preferably, the diseases arise from poorly differentiated acute leukemias, e.g., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L. (1991) *Crit Rev. in Oncol.*/*Hemotol.* 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

The 69624 nucleic acid and protein of the invention can be used to treat and/or diagnose a variety of immune, e.g., inflammatory, disorders. Examples of immune disorders or diseases include, but are not limited to, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy. Examples of inflammatory disorders include, but are not limited to, respiratory inflammatory disorders, chronic obstructive pulmonary disease, and inflammatory bowel disease.

The 69624 nucleic acid and protein of the invention can be used to treat and/or diagnose a variety of cardiovascular disorders. As used herein, disorders involving the heart, or "cardiovascular disease" or a "cardiovascular disorder" includes a disease or disorder which affects the cardiovascular system, e.g., the heart, the blood vessels, and/or the blood. A cardiovascular disorder can be caused by an imbalance in arterial pressure, a malfunction of the heart, or an occlusion of a blood vessel, e.g., by a thrombus. A cardiovascular disorder includes, but is not limited to disorders such as arteriosclerosis, atherosclerosis, cardiac hypertrophy, ischemia reperfusion injury, restenosis, arterial inflammation, vascular wall remodeling, ventricular remodeling, rapid ventricular pacing, coronary microembolism, tachycardia, bradycardia, pressure overload, aortic bending, coronary artery ligation, vascular heart disease, valvular disease, including but not limited to, valvular degeneration caused by calcification, rheumatic heart disease, endocarditis, or complications of artificial valves; atrial fibrillation, long-QT syndrome, congestive heart failure, sinus node dysfunction, angina, heart failure, hypertension, atrial fibrillation, atrial flutter, pericardial disease, including but not limited to, pericardial effusion and pericarditis; cardiomyopathies, *e.g.,* dilated cardiomyopathy or idiopathic cardiomyopathy, myocardial infarction, coronary artery disease, coronary artery spasm, ischemic disease, arrhythmia, sudden cardiac death, and cardiovascular developmental disorders (*e.g*., arteriovenous malformations, arteriovenous fistulae, raynaud's syndrome, neurogenic thoracic outlet syndrome, causalgia/reflex sympathetic dystrophy, hemangioma, aneurysm, cavernous angioma, aortic valve stenosis, atrial septal defects, atrioventricular canal, coarctation of the aorta, ebsteins anomaly, hypoplastic left heart syndrome, interruption of the aortic arch, mitral valve prolapse, ductus arteriosus, patent foramen ovale, partial anomalous pulmonary venous return, pulmonary atresia with ventricular septal defect, pulmonary atresia without ventricular septal defect, persistance of the fetal circulation, pulmonary valve stenosis, single ventricle, total anomalous pulmonary venous return, transposition of the great vessels, tricuspid atresia, truncus arteriosus, ventricular septal defects). A cardiovasular disease or disorder also can include an endothelial cell disorder.

The 69624 nucleic acid and protein of the invention can be used to treat and/or diagnose a variety of endothelial cell disorders. As used herein, an "endothelial cell disorder" includes a disorder characterized by aberrant, unregulated, or unwanted endothelial cell activity, *e.g.,* proliferation, migration, angiogenesis, or vascularization; or aberrant expression of cell surface adhesion molecules or genes associated with angiogenesis, *e.g.,* TIE-2, FLT and FLK. Endothelial cell disorders include tumorigenesis, tumor metastasis, psoriasis, diabetic retinopathy, endometriosis, Grave's disease, ischemic disease (*e.g*., atherosclerosis), and chronic inflammatory diseases *(e.g.,* rheumatoid arthritis).

Additionally, 69624 may play an important role in the regulation of pain disorders. Examples of pain disorders include, but are not limited to, pain response elicited during various forms of tissue injury, e.g., inflammation, infection, and ischemia, usually referred to as hyperalgesia (described in, for example, Fields, H.L. (1987) *Pain,* New York:McGraw-Hill); pain associated with musculoskeletal disorders, e.g., joint pain; tooth pain; headaches; pain associated with surgery; pain related to irritable bowel syndrome; or chest pain.

As discussed, successful treatment of 69624 disorders can be brought about by techniques that serve to inhibit the expression or activity of target gene products. For example, compounds, e.g., an agent identified using an assays described above, that proves to exhibit negative modulatory activity, can be used in accordance with the invention to prevent and/or ameliorate symptoms of 69624 disorders. Such molecules can include, but are not limited to peptides, phosphopeptides, small organic or inorganic molecules, or antibodies (including, for example, polyclonal, monoclonal, humanized, anti-idiotypic, chimeric or single chain antibodies, and Fab, F(ab')₂ and Fab expression library fragments, scFV molecules, and epitope-binding fragments thereof).

Further, antisense and ribozyme molecules that inhibit expression of the target gene can also be used in accordance with the invention to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extracellular protein, it can be preferable to co-administer normal target gene protein into the cell or tissue in order to maintain the requisite level of cellular or tissue target gene activity.

Another method by which nucleic acid molecules may be utilized in treating or preventing a disease characterized by 69624 expression is through the use of aptamer molecules specific for 69624 protein. Aptamers are nucleic acid molecules having a tertiary structure which permits them to specifically bind to protein ligands (see, e.g., Osborne, *et al.* (1997) *Curr. Opin. Chem Biol.* 1: 5-9; and Patel, D.J. (1997) *Curr Opin Chem Biol* 1:32-46). Since nucleic acid molecules may in many cases be more conveniently introduced into target cells than therapeutic protein molecules may be, aptamers offer a method by which 69624 protein activity may be specifically decreased without the introduction of drugs or other molecules which may have pluripotent effects.

Antibodies can be generated that are both specific for target gene product and that reduce target gene product activity. Such antibodies may, therefore, by administered in instances whereby negative modulatory techniques are appropriate for the treatment of 69624 disorders. For a description of antibodies, see the Antibody section above.

In circumstances wherein injection of an animal or a human subject with a 69624 protein or epitope for stimulating antibody production is harmful to the subject, it is possible to generate an immune response against 69624 through the use of anti-idiotypic antibodies (see, for example, Herlyn, D. (1999) *Ann Med* 31:66-78; and Bhattacharya-Chatterjee, M., and Foon, K.A. (1998) *Cancer Treat Res.* 94:51-68). If an anti-idiotypic antibody is introduced into a mammal or human subject, it should stimulate the production of anti-anti-idiotypic antibodies, which should be specific to the 69624 protein. Vaccines directed to a disease characterized by 69624 expression may also be generated in this fashion.

In instances where the target antigen is intracellular and whole antibodies are used, internalizing antibodies may be preferred. Lipofectin or liposomes can be used to deliver the antibody or a fragment of the Fab region that binds to the target antigen into cells. Where fragments of the antibody are used, the smallest inhibitory fragment that binds to the target antigen is preferred. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see e.g., Marasco *et al.* (1993) *Proc. Natl. Acad. Sci. USA* 90:7889-7893).

The identified compounds that inhibit target gene expression, synthesis and/or activity can be administered to a patient at therapeutically effective doses to prevent, treat or ameliorate 69624 disorders. A therapeutically effective dose refers to that amount of the compound sufficient to result in amelioration of symptoms of the disorders. Toxicity and therapeutic efficacy of such compounds can be determined by standard pharmaceutical procedures as described above.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another example of determination of effective dose for an individual is the ability to directly assay levels of "free" and "bound" compound in the serum of the test subject. Such assays may utilize antibody mimics and/or "biosensors" that have been created through molecular imprinting techniques. The compound which is able to modulate 69624 activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique can be seen in Ansell, R. J. *et al* (1996) *Current Opinion in Biotechnology* 7:89-94 and in Shea, K.J. (1994) *Trends in Polymer Science* 2:166-173. Such "imprinted" affinity matrixes are amenable to ligand-binding assays, whereby the immobilized monoclonal antibody component is replaced by an appropriately imprinted matrix. An example of the use of such matrixes in this way can be seen in Vlatakis, G. *et al* (1993) *Nature* 361:645-647. Through the use of isotope-labeling, the "free" concentration of compound which modulates the expression or activity of 69624 can be readily monitored and used in calculations of IC₅₀.

Such "imprinted" affinity matrixes can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual IC₅₀. An rudimentary example of such a "biosensor" is discussed in Kriz, D. *et al* (1995) *Analytical Chemistry* 67:2142-2144.

Another aspect of the invention pertains to methods of modulating 69624 expression or activity for therapeutic purposes. Accordingly, in an exemplary embodiment, the modulatory method of the invention involves contacting a cell with a 69624 or agent that modulates one or more of the activities of 69624 protein activity associated with the cell. An agent that modulates 69624 protein activity can be an agent as described herein, such as a nucleic acid or a protein, a naturally-occurring target molecule of a 69624 protein (e.g., a 69624 substrate or receptor), a 69624 antibody, a 69624 agonist or antagonist, a peptidomimetic of a 69624 agonist or antagonist, or other small molecule.

In one embodiment, the agent stimulates one or 69624 activities. Examples of such stimulatory agents include active 69624 protein and a nucleic acid molecule encoding 69624. In another embodiment, the agent inhibits one or more 69624 activities. Examples of such inhibitory agents include antisense 69624 nucleic acid molecules, anti-69624 antibodies, and 69624 inhibitors. These modulatory methods can be performed *in vitro* (e.g., by culturing the cell with the agent) or, alternatively, *in vivo* (e.g., by administering the agent to a subject). As such, the present invention provides methods of treating an individual afflicted with a disease or disorder characterized by aberrant or unwanted expression or activity of a 69624 protein or nucleic acid molecule. In one embodiment, the method involves administering an agent (e.g., an agent identified by a screening assay described herein), or combination of agents that modulates (e.g., up regulates or down regulates) 69624 expression or activity. In another embodiment, the method involves administering a 69624 protein or nucleic acid molecule as therapy to compensate for reduced, aberrant, or unwanted 69624 expression or activity.

Stimulation of 69624 activity is desirable in situations in which 69624 is abnormally downregulated and/or in which increased 69624 activity is likely to have a beneficial effect. For example, stimulation of 69624 activity is desirable in situations in which a 69624 is downregulated and/or in which increased 69624 activity is likely to have a beneficial effect. Likewise, inhibition of 69624 activity is desirable in situations in which 69624 is abnormally upregulated and/or in which decreased 69624 activity is likely to have a beneficial effect.

### Pharmacogenomics

The 69624 molecules of the present invention, as well as agents, or modulators which have a stimulatory or inhibitory effect on 69624 activity (e.g., 69624 gene expression) as identified by a screening assay described herein can be administered to individuals to treat (prophylactically or therapeutically) 69624 associated disorders (e.g., renal, hepatic, or neural disorders) associated with aberrant or unwanted 69624 activity. In conjunction with such treatment, pharmacogenomics (i.e., the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a 69624 molecule or 69624 modulator as well as tailoring the dosage and/or therapeutic regimen of treatment with a 69624 molecule or 69624 modulator.

Pharmacogenomics deals with clinically significant hereditary variations in the response to drugs due to altered drug disposition and abnormal action in affected persons. See, for example, Eichelbaum, M. *et al.* (1996) *Clin. Exp. Pharmacol. Physiol.* 23:983-985 and Linder, M.W. *et al.* (1997) *Clin. Chem.* 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (e.g., a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Alternatively, a method termed the "candidate gene approach," can be utilized to identify genes that predict drug response. According to this method, if a gene that encodes a drug's target is known (e.g., a 69624 protein of the present invention), all common variants of that gene can be fairly easily identified in the population and it can be determined if having one version of the gene versus another is associated with a particular drug response.

Alternatively, a method termed the "gene expression profiling," can be utilized to identify genes that predict drug response. For example, the gene expression of an animal dosed with a drug (e.g., a 69624 molecule or 69624 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a subject with a 69624 molecule or 69624 modulator, such as a modulator identified by one of the exemplary screening assays described herein.

The present invention further provides methods for identifying new agents, or combinations, that are based on identifying agents that modulate the activity of one or more of the gene products encoded by one or more of the 69624 genes of the present invention, wherein these products may be associated with resistance of the cells to a therapeutic agent. Specifically, the activity of the proteins encoded by the 69624 genes of the present invention can be used as a basis for identifying agents for overcoming agent resistance. By blocking the activity of one or more of the resistance proteins, target cells, e.g., human cells, will become sensitive to treatment with an agent that the unmodified target cells were resistant to.

Monitoring the influence of agents (e.g., drugs) on the expression or activity of a 69624 protein can be applied in clinical trials. For example, the effectiveness of an agent determined by a screening assay as described herein to increase 69624 gene expression, protein levels, or upregulate 69624 activity, can be monitored in clinical trials of subjects exhibiting decreased 69624 gene expression, protein levels, or downregulated 69624 activity. Alternatively, the effectiveness of an agent determined by a screening assay to decrease 69624 gene expression, protein levels, or downregulate 69624 activity, can be monitored in clinical trials of subjects exhibiting increased 69624 gene expression, protein levels, or upregulated 69624 activity. In such clinical trials, the expression or activity of a 69624 gene, and preferably, other genes that have been implicated in, for example, a 69624-associated disorder can be used as a "read out" or markers of the phenotype of a particular cell.

### 69624 Informatics

The sequence of a 69624 molecule is provided in a variety of media to facilitate use thereof. A sequence can be provided as a manufacture, other than an isolated nucleic acid or amino acid molecule, which contains a 69624. Such a manufacture can provide a nucleotide or amino acid sequence, e.g., an open reading frame, in a form which allows examination of the manufacture using means not directly applicable to examining the nucleotide or amino acid sequences, or a subset thereof, as they exists in nature or in purified form. The sequence information can include, but is not limited to, 69624 full-length nucleotide and/or amino acid sequences, partial nucleotide and/or amino acid sequences, polymorphic sequences including single nucleotide polymorphisms (SNPs), epitope sequence, and the like. In a preferred embodiment, the manufacture is a machine-readable medium, e.g., a magnetic, optical, chemical or mechanical information storage device.

As used herein, "machine-readable media" refers to any medium that can be read and accessed directly by a machine, e.g., a digital computer or analogue computer. Non-limiting examples of a computer include a desktop PC, laptop, mainframe, server (e.g., a web server, network server, or server farm), handheld digital assistant, pager, mobile telephone, and the like. The computer can be stand-alone or connected to a communications network, e.g., a local area network (such as a VPN or intranet), a wide area network (e.g., an Extranet or the Internet), or a telephone network (e.g., a wireless, DSL, or ISDN network). Machine-readable media include, but are not limited to: magnetic storage media, such as floppy discs, hard disc storage medium, and magnetic tape; optical storage media such as CD-ROM; electrical storage media such as RAM, ROM, EPROM, EEPROM, flash memory, and the like; and hybrids of these categories such as magnetic/optical storage media.

A variety of data storage structures are available to a skilled artisan for creating a machine-readable medium having recorded thereon a nucleotide or amino acid sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. The skilled artisan can readily adapt any number of data processor structuring formats (*e.g*., text file or database) in order to obtain computer readable medium having recorded thereon the nucleotide sequence information of the present invention.

In a preferred embodiment, the sequence information is stored in a relational database (such as Sybase or Oracle). The database can have a first table for storing sequence (nucleic acid and/or amino acid sequence) information. The sequence information can be stored in one field (e.g., a first column) of a table row and an identifier for the sequence can be store in another field (e.g., a second column) of the table row. The database can have a second table, e.g., storing annotations. The second table can have a field for the sequence identifier, a field for a descriptor or annotation text (e.g., the descriptor can refer to a functionality of the sequence, a field for the initial position in the sequence to which the annotation refers, and a field for the ultimate position in the sequence to which the annotation refers. Non-limiting examples for annotation to nucleic acid sequences include polymorphisms (e.g., SNP's) translational regulatory sites and splice junctions. Non-limiting examples for annotations to amino acid sequence include polypeptide domains, e.g., a domain described herein; active sites and other functional amino acids; and modification sites.

By providing the nucleotide or amino acid sequences of the invention in computer readable form, the skilled artisan can routinely access the sequence information for a variety of purposes. For example, one skilled in the art can use the nucleotide or amino acid sequences of the invention in computer readable form to compare a target sequence or target structural motif with the sequence information stored within the data storage means. A search is used to identify fragments or regions of the sequences of the invention which match a particular target sequence or target motif. The search can be a BLAST search or other routine sequence comparison, e.g., a search described herein.

Thus, in one aspect, the invention features a method of analyzing 69624, e.g., analyzing structure, function, or relatedness to one or more other nucleic acid or amino acid sequences. The method includes: providing a 69624 nucleic acid or amino acid sequence; comparing the 69624 sequence with a second sequence, e.g., one or more preferably a plurality of sequences from a collection of sequences, e.g., a nucleic acid or protein sequence database to thereby analyze 69624. The method can be performed in a machine, e.g., a computer, or manually by a skilled artisan.

The method can include evaluating the sequence identity between a 69624 sequence and a database sequence. The method can be performed by accessing the database at a second site, e.g., over the Internet.

As used herein, a "target sequence" can be any DNA or amino acid sequence of six or more nucleotides or two or more amino acids. A skilled artisan can readily recognize that the longer a target sequence is, the less likely a target sequence will be present as a random occurrence in the database. Typical sequence lengths of a target sequence are from about 10 to 100 amino acids or from about 30 to 300 nucleotide residues. However, it is well recognized that commercially important fragments, such as sequence fragments involved in gene expression and protein processing, may be of shorter length.

Computer software is publicly available which allows a skilled artisan to access sequence information provided in a computer readable medium for analysis and comparison to other sequences. A variety of known algorithms are disclosed publicly and a variety of commercially available software for conducting search means are and can be used in the computer-based systems of the present invention. Examples of such software include, but are not limited to, MacPattern (EMBL), BLASTN and BLASTX (NCBI).

Thus, the invention features a method of making a computer readable record of a sequence of a 69624 sequence which includes recording the sequence on a computer readable matrix. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention features, a method of analyzing a sequence. The method includes: providing a 69624 sequence, or record, in machine-readable form; comparing a second sequence to the 69624 sequence; thereby analyzing a sequence. Comparison can include comparing to sequences for sequence identity or determining if one sequence is included within the other, e.g., determining if the 69624 sequence includes a sequence being compared. In a preferred embodiment the 69624 or second sequence is stored on a first computer, e.g., at a first site and the comparison is performed, read, or recorded on a second computer, e.g., at a second site. E.g., the 69624 or second sequence can be stored in a public or proprietary database in one computer, and the results of the comparison performed, read, or recorded on a second computer. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention provides a machine-readable medium for holding instructions for performing a method for determining whether a subject has a 69624-associated disease or disorder or a pre-disposition to a 69624-associated disease or disorder, wherein the method comprises the steps of determining 69624 sequence information associated with the subject and based on the 69624 sequence information, determining whether the subject has a 69624-associated disease or disorder or a pre-disposition to a 69624-associated disease or disorder and/or recommending a particular treatment for the disease, disorder or pre-disease condition.

The invention further provides in an electronic system and/or in a network, a method for determining whether a subject has a 69624-associated disease or disorder or a pre-disposition to a disease associated with a 69624 wherein the method comprises the steps of determining 69624 sequence information associated with the subject, and based on the 69624 sequence information, determining whether the subject has a 69624-associated disease or disorder or a pre-disposition to a 69624-associated disease or disorder, and/or recommending a particular treatment for the disease, disorder or pre-disease condition. In a preferred embodiment, the method further includes the step of receiving information, e.g., phenotypic or genotypic information, associated with the subject and/or acquiring from a network phenotypic information associated with the subject. The information can be stored in a database, e.g., a relational database. In another embodiment, the method further includes accessing the database, e.g., for records relating to other subjects, comparing the 69624 sequence of the subject to the 69624 sequences in the database to thereby determine whether the subject as a 69624-associated disease or disorder, or a pre-disposition for such.

The present invention also provides in a network, a method for determining whether a subject has a 69624 associated disease or disorder or a pre-disposition to a 69624-associated disease or disorder associated with 69624, said method comprising the steps of receiving 69624 sequence information from the subject and/or information related thereto, receiving phenotypic information associated with the subject, acquiring information from the network corresponding to 69624 and/or corresponding to a 69624-associated disease or disorder (e.g., a renal, hepatic, or neural disorder), and based on one or more of the phenotypic information, the 69624 information (*e.g*., sequence information and/or information related thereto), and the acquired information, determining whether the subject has a 69624-associated disease or disorder or a pre-disposition to a 69624-associated disease or disorder. The method may further comprise the step of recommending a particular treatment for the disease, disorder or pre-disease condition.

The present invention also provides a method for determining whether a subject has a 69624 -associated disease or disorder or a pre-disposition to a 69624-associated disease or disorder, said method comprising the steps of receiving information related to 69624 (*e.g*., sequence information and/or information related thereto), receiving phenotypic information associated with the subject, acquiring information from the network related to 69624 and/or related to a 69624-associated disease or disorder, and based on one or more of the phenotypic information, the 69624 information, and the acquired information, determining whether the subject has a 69624-associated disease or disorder or a pre-disposition to a 69624-associated disease or disorder. The method may further comprise the step of recommending a particular treatment for the disease, disorder or pre-disease condition.

The contents of all references, patents and published patent applications cited throughout this application are incorporated herein by reference.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An isolated nucleic acid molecule selected from the group consisting of:
a) a nucleic acid molecule comprising a nucleotide sequence of SEQ ID NO:1 or, SEQ ID NO:3;
b) a nucleic acid molecule which encodes a polypeptide comprising the amino acid sequence of SEQ ID NO:2;
c) a nucleic acid molecule which encodes an antigenic peptide or fragment of a polypeptide comprising the amino acid sequence of SEQ ID NO:2, wherein the fragment comprises at least 8 contiguous amino acids of SEQ ID NO: 2; and
d) a nucleic acid molecule which encodes a naturally occurring allelic variant of a polypeptide comprising the amino acid sequence of SEQ ID NO:2, wherein the nucleic acid molecule hybridizes to a nucleic acid molecule comprising SEQ ID NO: 1, 3, or a complement thereof, under stringent conditions.

2. The isolated nucleic acid molecule of claim 1, which is selected from the group consisting of:
a) a nucleic acid comprising the nucleotide sequence of SEQ ID NO: 1 or SEQ ID NO:3; and
b) a nucleic acid molecule which encodes a polypeptide comprising the amino acid sequence of SEQ ID NO:2.

3. The nucleic acid molecule of claim 1 or 2, further comprising vector nucleic acid sequences.

4. The nucleic acid molecule of claim 1, 2 or 3, further comprising nucleic acid sequences encoding a heterologous polypeptide.

5. A host cell which contains the nucleic acid molecule of any of claims 1 to 4.

6. The host cell of claim 5 which is a mammalian host cell.

7. A non-human mammalian host cell containing the nucleic acid molecule of any of claims 1 to 4.

8. An isolated polypeptide selected from the group consisting of:
a) a polypeptide which is encoded by a nucleic acid molecule comprising the nucleotide sequence of SEQ ID NO: 1, SEQ ID NO:3, or a complement thereof;
b) a naturally occurring allelic variant of a polypeptide comprising the amino acid sequence of SEQ ID NO:2, wherein the polypeptide is encoded by a nucleic acid molecule which hybridizes to a nucleic acid molecule comprising SEQ ID NO: 1, SEQ ID NO:3, or a complement thereof under stringent conditions; and
c) an antigenic peptide or fragment of a polypeptide comprising the amino acid sequence of SEQ ID NO:2, wherein the fragment comprises at least 8 contiguous amino acids of SEQ ID NO:2.

9. The isolated polypeptide of claim 8 comprising the amino acid sequence of SEQ ID NO:2.

10. The polypeptide of claim 8 or 9, further comprising heterologous amino acid sequences.

11. An antibody which selectively binds to a polypeptide of claim 8, 9 or 10.

12. A method for producing a polypeptide selected from the group consisting of:
a) a polypeptide comprising the amino acid sequence of SEQ ID NO:2;
b) a polypeptide comprising an antigenic peptide or fragment of the amino acid sequence of SEQ ID NO:2, wherein the fragment comprises at least 8 contiguous amino acids of SEQ ID NO:2; and
c) a naturally occurring allelic variant of a polypeptide comprising the amino acid sequence of SEQ ID NO:2, wherein the polypeptide is encoded by a nucleic acid molecule which hybridizes to a nucleic acid molecule comprising SEQ ID NO:1, SEQ ID NO:3, or a complement thereof under stringent conditions;
comprising culturing the host cell of claim 5, 6 or 7 under conditions in which the nucleic acid molecule is expressed.

13. A method for detecting the presence of a polypeptide of claim 8, 9 or 10 in a sample, comprising:
a) contacting the sample with a compound which selectively binds to a polypeptide of claim 8, 9 or 10, as the case may be; and
b) determining whether the compound binds to the polypeptide in the sample.

14. The method of claim 13, wherein the compound which binds to the polypeptide is an antibody.

15. A kit comprising a compound which selectively binds to a polypeptide of claim 8, 9 or 10 and instructions for use.

16. A method for detecting the presence of a nucleic acid molecule of any of claims 1 to 4 in a sample, comprising the steps of:
a) contacting the sample with a nucleic acid probe or primer which selectively hybridizes to the nucleic acid molecule; and
b) determining whether the nucleic acid probe or primer binds to a nucleic acid molecule in the sample.

17. The method of claim 16, wherein the sample comprises mRNA molecules and is contacted with a nucleic acid probe.

18. A kit comprising a compound which selectively hybridizes to a nucleic acid molecule of any of claims 1 to 4, and instructions for use.

19. A method for identifying a compound which binds to a polypeptide of claim 8, 9 or 10, comprising the steps of:
a) contacting a polypeptide, or a cell expressing a polypeptide of claim 8, 9 or 10, as the case may be, with a test compound; and
b) determining whether the polypeptide binds to the test compound.

20. The method of claim 19, wherein the binding of the test compound to the polypeptide is detected by a method selected from the group consisting of:
a) detection of binding by direct detecting of test compound/polypeptide binding;
b) detection of binding using a competition binding assay; and
c) detection of binding using an assay for 69624-mediated signal transduction.

21. A method for modulating the activity of a polypeptide of claim 8, 9 or 10, comprising contacting a polypeptide or a cell expressing a polypeptide of claim 8, 9 or 10, as the case may be, with a compound which binds to the polypeptide in a sufficient concentration to modulate the activity of the polypeptide.

22. A method for identifying a compound which modulates the activity of a polypeptide of claim 8, 9 or 10, comprising:
a) contacting a polypeptide of claim 8, 9 or 10, as the case may be, with a test compound; and
b) determining the effect of the test compound on the activity of the polypeptide to thereby identify a compound which modulates the activity of the polypeptide.


SEQUENCE LISTING

<110> Curtis, Rory A.J.
      Millennium Pharmaceuticals, Inc.

<120> 69624, A Human Transporter Family Member
  and Uses Therefor

<130> P71982EP

<150> 60/298,970
<151> 2001-06-18

<160> 8

<170> FastSEQ for Windows Version 4.0

<210> 1
<211> 2534
<212> DNA
<213> Homo sapiens

<220>
<221> CDS
<222> (12)...(1718)

<400> 1
cctcccgcgc g atg gcc tcg gcg ctg agc tat gtc tcc aag ttc aag tcc   50
ttc gtg atc ttg ttc gtc acc ccg ctc ctg ctg ctg cca ctc gtc att   98
ctg atg ccc gcc aag ttt gtc agg tgt gcc tac gtc atc atc ctc atg   146
gcc att tac tgg tgc aca gaa gtc atc cct ctg gct gtc acc tct ctc   194
atg cct gtc ttg ctt ttc cca ctc ttc cag att ctg gac tcc agg cag   242
gtg tgt gtc cag tac atg aag gac acc aac atg ctg ttc ctg ggc ggc   290
ctc atc gtg gcc gtg gct gtg gag cgc tgg aac ctg cac aag agg atc   338
gcc ctg cgc acg ctc ctc tgg gtg ggg gcc aag cct gca cgg ctg atg   386
ctg ggc ttc atg ggc gtc aca gcc ctc ctg tcc atg tgg atc agt aac   434
acg gca acc acg gcc atg atg gtg ccc atc gtg gag gcc ata ttg cag     482
cag atg gaa gcc aca agc gca gcc acc gag gcc ggc ctg gag ctg gtg     530
gac aag ggc aag gcc aag gag ctg cca ggg agt caa gtg att ttt gaa     578
ggc ccc act ctg ggg cag cag gaa gac caa gag cgg aag agg ttg tgt     626
aag gcc atg acc ctg tgc atc tgc tac gcg gcc agc atc ggg ggc acc     674
gcc acc ctg acc ggg acg gga ccc aac gtg gtg ctc ctg ggc cag atg     722
aac gag ttg ttt cct gac agc aag gac ctc gtg aac ttt gct tcc tgg     770
ttt gca ttt gcc ttt ccc aac atg ctg gtg atg ctg ctg ttc gcc tgg     818
ctg tgg ctc cag ttt gtt tac atg aga ttc aat ttt aaa aag tcc tgg     866
ggc tgc ggg cta gag agc aag aaa aac gag aag gct gcc ctc aag gtg     914
ctg cag gag gag tac cgg aag ctg ggg ccc ttg tcc ttc gcg gag atc     962
aac gtg ctg atc tgc ttc ttc ctg ctg gtc atc ctg tgg ttc tcc cga     1010
gac ccc ggc ttc atg ccc ggc tgg ctg act gtt gcc tgg gtg gag ggt     1058
gag aca aag tat gtc tcc gat gcc act gtg gcc atc ttt gtg gcc acc     1106
ctg cta ttc att gtg cct tca cag aag ccc aag ttt aac ttc cgc agc     1154
cag act gag gaa gaa agg aaa act cca ttt tat ccc cct ccc ctg ctg 1202
gat tgg aag gta acc cag gag aaa gtg ccc tgg ggc atc gtg ctg cta 1250
cta ggg ggc gga ttt gct ctg gct aaa gga tcc gag gcc tcg ggg ctg 1298
tcc gtg tgg atg ggg aag cag atg gag ccc ttg cac gca gtg ccc ccg 1346
gca gcc atc acc ttg atc ttg tcc ttg ctc gtt gcc gtg ttc act gag 1394
tgc aca agc aac gtg gcc acc acc acc ttg ttc ctg ccc atc ttt gcc 1442
tcc atg tct cgc tcc atc ggc ctc aat ccg ctg tac atc atg ctg ccc 1490
tgt acc ctg agt gcc tcc ttt gcc ttc atg ttg cct gtg gcc acc cct 1538
cca aat gcc atc gtg ttc acc tat ggg cac ctc aag gtt gct gac atg 1586
gtg aaa aca gga gtc ata atg aac ata att gga gtc ttc tgt gtg ttt 1634
ttg gct gtc aac acc tgg gga cgg gcc ata ttt gac ttg gat cat ttc 1682
cct gac tgg gct aat gtg aca cat att gag act tag gaagagccac 1728
aagaccacac acacagccct taccctcctc aggactaccg aaccttctgg cacaccttgt 1788
acagagtttt ggggttcaca ccccaaaatg acccaacgat gtccacacac caccaaaacc 1848
cagccaatgg gccacctctt cctccaagcc cagatgcaga gatggtcatg ggcagctgga 1908
gggtaggctc agaaatgaag ggaacccctc agtgggctgc tggacccatc tttcccaagc 1968
ctcacctgag gcccagagtg gacacagctg ttaatttcca ctggctatgc cacttcagag 2028
tctttcatgc cagcgtttga gctcctctgg gtaaaatctt ccctttgttg actggccttc 2088
acagccatgg ctggtgacaa cagaggatcg ttgagattga gcagcgcttg gtgatctctc 2148
agcaaacaac ccctgcccgt gggccaatct acttgaagtt actcggacaa agaccccaaa 2208
gtggggcaac aactccagag aggctgtggg aatcttcaga agcccccctg taagagacag 2268
acatgagaga caagcatctt ctttcccccg caagtccatt ttatttcctt cttgtgctgc 2328
tctggaagag aggcagtagc aaagagatga gctcctggat ggcattttcc agggcaggag 2388
aaagtatgag agcctcagga aaccccatca aggaccgagt atgtgtctgg ttccttgggt 2448
gggacgattc ctgaccacac tgtccagctc ttgctctcat taaatgctct gtctcccgcg 2508
gaaaaaaaaa aaaaaagggc ggccgc 2534

<210> 2
<211> 568
<212> PRT
<213> Homo sapiens

<400> 2


<210> 3
<211> 1707
<212> DNA
<213> Homo sapiens

<220>
<221> CDS
<222> (1)...(1707)

<400> 3
atg gcc tcg gcg ctg agc tat gtc tcc aag ttc aag tcc ttc gtg atc      48
ttg ttc gtc acc ccg ctc ctg ctg ctg cca ctc gtc att ctg atg ccc      96
gcc aag ttt gtc agg tgt gcc tac gtc atc atc ctc atg gcc att tac     144
tgg tgc aca gaa gtc atc cct ctg gct gtc acc tct ctc atg cct gtc     192
ttg ctt ttc cca ctc ttc cag att ctg gac tcc agg cag gtg tgt gtc     240
cag tac atg aag gac acc aac atg ctg ttc ctg ggc ggc ctc atc gtg     288
gcc gtg gct gtg gag cgc tgg aac ctg cac aag agg atc gcc ctg cgc     336
acg ctc ctc tgg gtg ggg gcc aag cct gca cgg ctg atg ctg ggc ttc 384
atg ggc gtc aca gcc ctc ctg tcc atg tgg atc agt aac acg gca acc 432
acg gcc atg atg gtg ccc atc gtg gag gcc ata ttg cag cag atg gaa 480
gcc aca agc gca gcc acc gag gcc ggc ctg gag ctg gtg gac aag ggc 528
aag gcc aag gag ctg cca ggg agt caa gtg att ttt gaa ggc ccc act 576
ctg ggg cag cag gaa gac caa gag cgg aag agg ttg tgt aag gcc atg 624
acc ctg tgc atc tgc tac gcg gcc agc atc ggg ggc acc gcc acc ctg 672
acc ggg acg gga ccc aac gtg gtg ctc ctg ggc cag atg aac gag ttg 720
ttt cct gac agc aag gac ctc gtg aac ttt gct tcc tgg ttt gca ttt 768
gcc ttt ccc aac atg ctg gtg atg ctg ctg ttc gcc tgg ctg tgg ctc 816
cag ttt gtt tac atg aga ttc aat ttt aaa aag tcc tgg ggc tgc ggg 864
cta gag agc aag aaa aac gag aag gct gcc ctc aag gtg ctg cag gag 912
gag tac cgg aag ctg ggg ccc ttg tcc ttc gcg gag atc aac gtg ctg 960
atc tgc ttc ttc ctg ctg gtc atc ctg tgg ttc tcc cga gac ccc ggc 1008
ttc atg ccc ggc tgg ctg act gtt gcc tgg gtg gag ggt gag aca aag 1056
tat gtc tcc gat gcc act gtg gcc atc ttt gtg gcc acc ctg cta ttc      1104
att gtg cct tca cag aag ccc aag ttt aac ttc cgc agc cag act gag      1152
gaa gaa agg aaa act cca ttt tat ccc cct ccc ctg ctg gat tgg aag      1200
gta acc cag gag aaa gtg ccc tgg ggc atc gtg ctg cta cta ggg ggc      1248
gga ttt gct ctg gct aaa gga tcc gag gcc tcg ggg ctg tcc gtg tgg      1296
atg ggg aag cag atg gag ccc ttg cac gca gtg ccc ccg gca gcc atc      1344
acc ttg atc ttg tcc ttg ctc gtt gcc gtg ttc act gag tgc aca agc      1392
aac gtg gcc acc acc acc ttg ttc ctg ccc atc ttt gcc tcc atg tct      1440
cgc tcc atc ggc ctc aat ccg ctg tac atc atg ctg ccc tgt acc ctg      1488
agt gcc tcc ttt gcc ttc atg ttg cct gtg gcc acc cct cca aat gcc      1536
atc gtg ttc acc tat ggg cac ctc aag gtt gct gac atg gtg aaa aca      1584
gga gtc ata atg aac ata att gga gtc ttc tgt gtg ttt ttg gct gtc      1632
aac acc tgg gga cgg gcc ata ttt gac ttg gat cat ttc cct gac tgg      1680
gct aat gtg aca cat att gag act tag                                  1707

<210> 4

<211> 585
<212> PRT
<213> Artificial Sequence

<220>
<223> consensus

<400> 4
Met Lys Lys Lys Leu Ile Trp Lys Leu Leu Ala Leu Leu Ala Val Leu
1               5                   10                  15
Leu Ile Ile Trp Leu Leu Pro Ala Pro Asp Gly Leu Ser Pro Lys Ala
            20                  25                  30
Trp His Leu Phe Ala Ile Phe Ile Ala Met Ile Val Gly Leu Ile Leu
        35                  40                  45
Glu Pro Val Pro Leu Gly Val Ile Ala Leu Ile Ala Ile Ser Leu Ser
    50                  55                  60
Ala Val Leu Thr Gly Thr Leu Ser Phe Glu Asn Glu Glu Leu Ala Gln
65                  70                  75                  80
Pro Gly Phe Lys Ala Thr Ala Lys Ala Leu Ser Trp Ala Leu Ser Gly
                85                  90                  95
Phe Ser Asn Thr Thr Thr Trp Leu Val Phe Gly Ala Phe Phe Leu Ala
            100                 105                 110
Ala Gly Phe Glu Lys Thr Gly Leu Gly Lys Arg Ile Ala Leu Val Leu
        115                 120                 125
Val Lys Ala Ile Gly Lys Arg Thr Ala Leu Leu Gly Leu Gly Tyr Ala
    130                 135                 140
Leu Val Phe Ser Asp Leu Leu Leu Ala Pro Ala Ile Pro Ser Asn Arg
145                 150                 155                 160
Thr Ala Arg Ala Gly Gly Ile Ile Phe Pro Val Ile Lys Ser Leu Pro
                165                 170                 175
Val Ser Gln Asn Val Glu Glu Gly Leu Phe Gly Ser Thr Pro Glu Lys
            180                 185                 190
Gly Glu Pro Ser Pro Gln Lys Glu Val Thr Lys Leu Asp Asn Gly Gln
        195                 200                 205
Ala Leu Pro Val Pro Ser Ala Pro Glu Asp Glu His Leu Thr Gln Glu
    210                 215                 220
Ser Glu Arg Lys Ile Gly Ala Tyr Leu Met Leu Thr Val Tyr Gln Ser
225                 230                 235                 240
Thr Ser Ile Thr Ser Ala Met Phe Leu Thr Ala Met Ala Pro Asn Leu
                245                 250                 255
Leu Leu Leu Gly Leu Met Asn Lys Leu Phe Pro Ile Leu Gly Asn Glu
            260                 265                 270
Ile Ser Trp Ala Ser Trp Phe Leu Ala Ala Leu Pro Pro Gly Ile Ile
        275                 280                 285
Leu Leu Leu Leu Val Pro Leu Leu Leu Tyr Val Leu Tyr Pro Pro Glu
    290                 295                 300
Ile Lys Ser Ser Pro Phe Gly Ile Gly Glu Lys Glu Glu Glu Gln Gln
305                 310                 315                 320
Gln Asp Ala Lys Arg Ile Ala Lys Thr Glu Leu Lys Glu Met Gly Pro
                325                 330                 335
Met Ser Phe Arg Glu Lys Thr Leu Leu Val Leu Phe Leu Leu Leu Leu
            340                 345                 350
Leu Leu Trp Ile Phe Gly Gly Pro Gly Phe Phe Leu Gly Trp Gly Asn
        355                 360                 365
Leu Val Ser Leu Gly Ala Lys Gly Glu Ser Met Ile Asp Ala Thr Thr
    370                 375                 380
Val Ala Ile Ile Val Leu Val Leu Leu Leu Leu Leu Arg Ser Lys Ile
385                 390                 395                 400
Pro Gly Leu Thr Gln Asp Pro Glu Asn Pro Gly Lys Leu Lys Ala Pro
                405                 410                 415
Pro Ala Ile Val Thr Trp Lys Lys Asp Ile Val Lys Asn Lys Thr Ala

420                 425                 430
Trp Asn Thr Leu Ile Trp Leu Gly Gly Leu Ile Ala Leu Ala Gly Gly
        435                 440                 445
Leu Asn Lys Ser Gly Phe Ile Glu Trp Leu Gly Asn Lys Leu Val Thr
    450                 455                 460
Leu Leu Ser Leu Ala Gly Leu Ser Pro Leu Met Ala Phe Ile Val Leu
465                 470                 475                 480
Ile Leu Leu Leu Tyr Leu Ser His Tyr Phe Phe Ala Ser Ala Thr Ala
                485                 490                 495
His Thr Ala Ala Met Leu Pro Ile Phe Leu Ala Val Ala Gln Ala Ile
            500                 505                 510
Pro Gly Ala Pro Pro Leu Leu Leu Ala Leu Leu Leu Ala Phe Ala Ser
        515                 520                 525
Ser Leu Gly Gly Phe Leu Thr Pro Tyr Gly Thr Gly Pro Asn Pro Ile
    530                 535                 540
Tyr Phe Gly Ser Gly Tyr Val Lys Val Lys Asp Trp Trp Arg Leu Gly
545                 550                 555                 560
Phe Ile Leu Ser Ile Ile Gly Leu Ile Ile Trp Leu Leu Val Gly Ala
                565                 570                 575
Leu Trp Trp Lys Ala Leu Gly Leu Trp
            580                 585

<210> 5
<211> 587
<212> PRT
<213> Rattus norvegicus

<400> 5
Met Ala Thr Cys Trp Pro Ala Leu Trp Ala Tyr Arg Phe Tyr Leu Ile
 1               5                  10                  15
Val Leu Cys Leu Pro Ile Phe Leu Leu Pro Leu Pro Leu Ile Val Gln
            20                  25                  30
Thr Lys Glu Ala Tyr Cys Ala Tyr Ser Ile Ile Leu Met Ala Leu Leu
        35                  40                  45
Trp Cys Thr Glu Ala Leu Pro Leu Ala Val Thr Ala Leu Phe Pro Ile
    50                  55                  60
Val Leu Phe Pro Leu Met Gly Ile Met Asp Ala Ser Glu Val Cys Ile
65                  70                  75                  80
Glu Tyr Phe Lys Asp Thr Asn Ile Leu Phe Val Gly Gly Leu Met Val
                85                  90                  95
Ala Ile Ala Val Glu His Trp Asn Leu His Lys Arg Ile Ala Leu Gln
            100                 105                 110
Val Leu Leu Ile Ile Gly Val Arg Pro Ala Leu Leu Leu Leu Gly Phe
        115                 120                 125
Met Leu Val Thr Ala Phe Leu Ser Met Trp Ile Ser Asn Thr Ala Thr
    130                 135                 140
Thr Ala Met Met Val Pro Ile Gly His Ala Val Leu Glu Gln Leu Gln
145                 150                 155                 160
Gly Ser Lys Lys Asp Val Glu Gly Gly Asn Asn Asn Pro Thr Phe Glu
                165                 170                 175
Leu Gln Glu Glu Cys Pro Gln Lys Glu Val Thr Lys Leu Asp Asn Gly
            180                 185                 190
Gln Pro Val Ser Ala Pro Ser Glu Pro Arg Thr Gln Lys Thr Gln Glu
        195                 200                 205
His His Arg Phe Ser Gln Gly Leu Ser Leu Cys Ile Cys Tyr Ser Ala
    210                 215                 220
Ser Ile Gly Gly Ile Ala Thr Leu Thr Gly Thr Thr Pro Asn Leu Val
225                 230                 235                 240
Leu Gln Gly Gln Val Asn Ser Leu Phe Pro Gln Asn Gly Asn Val Val
                245                 250                 255

Asn Phe Ala Ser Trp Phe Gly Phe Ala Phe Pro Thr Met Ile Ile Leu
            260                 265                 270
Leu Leu Leu Ala Trp Leu Trp Leu Gln Val Leu Phe Leu Gly Val Asn
        275                 280                 285
Phe Arg Lys Asn Phe Gly Phe Gly Glu Gly Glu Glu Glu Arg Lys Gln
    290                 295                 300
Ala Ala Phe Gln Val Ile Lys Thr Gln Tyr Arg Leu Leu Gly Pro Met
305                 310                 315                 320
Ser Phe Ala Glu Lys Thr Val Thr Val Leu Phe Val Leu Leu Val Val
                325                 330                 335
Leu Trp Phe Thr Arg Glu Pro Gly Phe Phe Pro Gly Trp Gly Asp Thr
            340                 345                 350
Val Phe Ala Asn Glu Lys Gly Gln Ser Met Ala Ser Asp Gly Thr Val
        355                 360                 365
Ala Ile Phe Ile Ser Leu Val Met Phe Ile Ile Pro Ser Lys Ile Pro
    370                 375                 380
Gly Leu Met Gln Asp Pro Lys Lys Pro Gly Lys Leu Lys Ala Pro Pro
385                 390                 395                 400
Ala Ile Leu Thr Trp Lys Thr Val Asn Asp Lys Met Pro Trp Asn Ile
                405                 410                 415
Val Ile Leu Leu Gly Gly Gly Phe Ala Leu Ala Lys Gly Ser Glu Gln
            420                 425                 430
Ser Gly Leu Ser Glu Trp Leu Gly Asp Lys Leu Thr Pro Leu Gln His
        435                 440                 445
Ile Pro Pro Ser Ala Thr Ala Val Ile Leu Cys Leu Leu Ile Ala Ile
    450                 455                 460
Phe Thr Glu Cys Thr Ser Asn Val Ala Thr Thr Thr Leu Phe Leu Pro
465                 470                 475                 480
Ile Leu Ala Ser Met Ala Gln Ala Ile Cys Leu His Pro Leu Tyr Val
                485                 490                 495
Met Leu Pro Cys Thr Leu Ala Ala Ser Leu Ala Phe Met Leu Pro Val
            500                 505                 510
Ala Thr Pro Pro Asn Ala Ile Val Phe Ser Phe Gly Gly Leu Lys Val
        515                 520                 525
Ser Asp Met Ala Arg Ala Gly Phe Leu Leu Asn Ile Ile Gly Val Leu
    530                 535                 540
Ala Ile Thr Leu Ser Ile Asn Ser Trp Ser Ile Pro Ile Phe Lys Leu
545                 550                 555                 560
Asp Thr Phe Pro Ser Trp Ala His Ser Asn Thr Ser Gln Cys Leu Leu
                565                 570                 575
Asn Pro Ser Asn Ser Thr Val Pro Gly Gly Leu
            580                 585


<210> 6
<211> 17
<212> PRT
<213> Artificial Sequence

<220>
<223> consensus

<221> VARIANT
<222> 1
<223> Xaa = Ser, Thr, Ala, Cys, or Pro

<221> VARIANT
<222> 9, 16
<223> Xaa = Leu, Ile, Val, or Met

<221> VARIANT

<222> 10
<223> Xaa = Gly, Ser, or Ala

<221> VARIANT
<222> 3, 4, 6, 7, 11-13, 15
<223> Xaa = any amino acid

<400> 6
Xaa Ser Xaa Xaa Phe Xaa Xaa Pro Xaa Xaa Xaa Xaa Xaa Asn Xaa Xaa
 1               5                   10                  15
Val

<210> 7
<211> 132
<212> PRT
<213> Artificial Sequence

<220>
<223> consensus

<400> 7
Val Ile Ile Leu Met Ala Leu Tyr Trp Cys Thr Glu Ala Leu Pro Leu
 1               5                   10                  15
Ala Val Thr Ala Leu Leu Pro Leu Ile Leu Phe Pro Leu Phe Gly Ile
            20                  25                  30
Met Lys Asp Ser Gly Ser Ser Ser Glu Glu Val Cys Ser Ser Tyr Phe
        35                  40                  45
Lys Asp Thr Ile Ile Leu Phe Leu Gly Gly Leu Ile Leu Ala Leu Ala
    50                  55                  60
Ile Glu Lys Trp Asn Leu His Lys Arg Ile Ala Leu Arg Ile Leu Leu
65                  70                  75                  80
Ile Val Gly Thr Lys Pro Ala Arg Leu Leu Leu Gly Phe Met Met Val
                85                  90                  95
Thr Ala Phe Leu Ser Met Trp Ile Ser Asn Thr Ala Thr Thr Ala Met
            100                 105                 110
Met Met Pro Ile Ala Gln Ala Val Leu Glu Glu Leu Glu Ser Ser Glx
        115                 120                 125
Lys Asp Val Glu
    130

<210> 8
<211> 177
<212> PRT
<213> Artificial Sequence

<220>
<223> consensus

<400> 8
Gln Lys Phe Ser Gln Gly Leu Met Leu Cys Ile Cys Tyr Ser Ala Ser
 1               5                   10                  15
Ile Gly Gly Leu Gly Thr Leu Thr Gly Thr Gly Pro Asn Leu Val Leu
            20                  25                  30
Gln Gly Gln Met Asn Ser Arg Phe Pro Glu Ser Cys Asp Glu Ile Asn
        35                  40                  45
Phe Ala Ser Trp Phe Phe Phe Ala Phe Pro Leu Met Leu Ile Leu Leu
    50                  55                  60
Leu Leu Ser Trp Leu Trp Leu Gln Trp Leu Tyr Leu Gly Leu Asn Phe

65                  70                  75                  80
Lys Lys Asn Phe Ser Cys Gln Lys Thr Lys Glu Glu Thr Lys Lys Glu
                85                  90                  95
Glu Ala Ala Lys Glu Val Ile Lys Gln Glu Tyr Glu Lys Leu Gly Pro
            100                 105                 110
Met Ser Phe Ala Glu Met Ser Val Leu Ile Leu Phe Val Leu Met Val
        115                 120                 125
Ile Leu Trp Phe Thr Arg Asp Pro Gly Phe Ile Pro Gly Trp Gly Asp
    130                 135                 140
Leu Phe Phe Ala Asn Glu Lys Gly Lys Gly Tyr Val Ser Asp Gly Thr
145                 150                 155                 160
Val Ala Ile Phe Ile Ser Ile Leu Leu Phe Ile Ile Pro Ser Lys Lys
                165                 170                 175
Pro